# EUROPEAN PATENT SPECIFICATION

(11) **EP 3 847 277 B1**
(45) Date of publication and mention of the grant of the patent: **11.12.2024**
(21) Application number: 19773589.7
(22) Date of filing: 05.09.2019
(51) Int. Cl.: C12Q 1/686, C12Q 1/689

(54) **SEQUENCES AND THEIR USE FOR DETECTION AND CHARACTERIZATION OF ESCHERICHIA COLI SEROTYPE O157:H7**
SEQUENZEN UND IHRE VERWENDUNG FÜR DEN NACHWEIS UND DIE CHARAKTERISIERUNG VON ESCHERICHIA COLI SEROTYP O157:H7
SÉQUENCES ET LEUR UTILISATION POUR LA DÉTECTION ET LA CARACTÉRISATION DE E. COLI O157:H7

(30) Priority: 06.09.2018 US 201862727755 P
(43) Date of publication of application: 14.07.2021
(73) Proprietor: Hygiena, LLC, Camarillo CA 93012 (US)
(72) Inventor: KALBURGE, Sai Siddarth, Claymont, DE 19703 (US); WANG, Yangyang, Bear, DE 19701 (US); PADMALAYAM, Indira, Wilmington, DE 19801 (US); EASTER, Martin, London WD5 0BP (GB)
(74) Representative: Dehns
(86) International application number: PCT/US2019/049699
(87) International publication number: WO 2020/051301

(56) References cited:
- WO-A1-02/36827
- WO-A2-2013/186754
- JP-A- 2002 355 074
- US-A1- 2011 020 823
- US-A1- 2011 165 568
- M. EPPINGER ET AL: "Genomic anatomy of Escherichia coli O157:H7 outbreaks", PROCEEDINGS OF THE NATIONAL ACADEMY OF SCIENCES (PNAS), vol. 108, no. 50, 30 November 2011 (2011-11-30), US, pages 20142 - 20147, XP055634385, ISSN: 0027-8424, DOI: 10.1073/pnas.1107176108

## Description

### CROSS-REFERENCE TO RELATED APPLICATIONS

This application claims priority to U.S. Provisional Application No. 62/727,755, filed on September 6, 2018.

### FIELD

The field relates to methods for detection and characterization of *Escherichia coli* bacteria serotype O157:H7 based on the presence of nucleic acid sequences, for example PCR-based methods for detection, and to oligonucleotide molecules and reagents and kits useful therefor.

### BACKGROUND

*Escherichia coli* (*E. coli*) is a gram-negative, rod-shaped bacterium. Most *E*. *coli* strains are benign and are part of the normal intestinal flora of humans and other animal. However, some strains of *E*. *coli* have acquired virulence factors and evolved into pathogens that can cause severe and sometimes-fatal disease. Pathogenic strains of *E. coli* are categorized based on virulence factors, mechanism of pathogenesis, clinical symptoms and/or serogroups, as defined by the 0 and H antigens.

Enterohemorrhagic *E*. *coli* (EHEC) are a category of *E*. *coli* strains that produce shiga toxins and cause hemorrhagic colitis or bloody diarrhea that can progress to life-threatening sequalae such as hemolytic uremic syndrome in humans. Of the several pathogenic serotypes of EHEC, the 0157:H7 serotype is one of the most frequently isolated EHEC strains from clinical cases.

The *E. coli* 0157:H7 serotype has been associated with several food and water borne outbreaks worldwide. Cattle are the major reservoir of *E*. *coli* O157:H7, and it is hence regulated as an adulterant in ground beef by the U.S. Department of Agriculture (USDA) with a zero-tolerance standard. Additionally, *E. coli* O157:H7 has also been isolated from fecal samples of sheep, goat, pigs and turkeys. Other contaminated foods that have been associated with outbreaks include unpasteurized milk, drinking water, salami, beef jerky, and fresh produce such as lettuce, radish sprouts, fresh spinach, and apple cider.

Considering the ubiquitous nature of *E*. *coli,* it is critical to be able to specifically detect the highly pathogenic and tightly regulated *E.* coli serotype 0157:H7 in food and environmental samples, even in the presence of other *E*. coli serotypes. Published U.S. patent application No. 2011/0020823 and Sharma (Mol. Cell. Probes 20:298-306 (2006)) describe a method for detecting *E. coli* O157:H7 through simultaneous amplification of two sequences which when amplified together in combination indicate the presence of *E*. *coli* O157:H7. However, while *E. coli* O157:H7 strains are the only group that contain both sequences, there are other strains of *E*. *coli* that contain one or the other of the two sequences. In a sample containing a mixture of two different *E*. *coli* strains that contain the two sequences, there is a potential to obtain erroneous results that can be falsely interpreted as being positive for the presence of *E. coli* O157:H7.

Therefore, it is desirable to have an assay that reduces the potential for false-positive results and accurately detects *E*. *coli* O157:H7 in a sample using a fast and easy-to-use detection system.

### SUMMARY

One aspect is for a method for detecting the presence of *E*. *coli* O157:H7 in a sample, said sample comprising nucleic acids, said method comprising:
(a) providing a reaction mixture comprising a primer pair selected from group consisting of primer pair SEQ ID NO:1 and SEQ ID NO:2, primer pair SEQ ID NO:3 and SEQ ID NO:4, primer pair SEQ ID NO:5 and SEQ ID NO:6, primer pair SEQ ID NO:7 and SEQ ID NO:8, and a combination thereof;
(b) performing PCR amplification of said nucleic acids of said sample using the reaction mixture of step (a); and
(c) detecting the amplification of step (b), whereby a positive detection of amplification indicates the presence of *E*. *coli* O157:H7 in the sample.

Another aspect is for an isolated polynucleotide comprising SEQ ID N0:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID N0:10, SEQ ID NO:11, or SEQ ID NO:12.

A further aspect is for a replication composition for use in performance of PCR, comprising:
(a) a primer pair selected from the group consisting of primer pair SEQ ID N0:1 and SEQ ID NO:2, primer pair SEQ ID NO:3 and SEQ ID NO:4, primer pair SEQ ID NO:5 and SEQ ID NO:6, primer pair SEQ ID NO:7 and SEQ ID NO:8, and a combination thereof; and
(b) thermostable DNA polymerase.

An additional aspect is for a kit for detection of *E*. *coli O157:H7*, inclusive of all serotypes, in a sample, comprising the aforementioned replication composition.

A further aspect is for a tablet comprising the aforementioned replication composition.

Another aspect is for a method for detecting the presence of *E*. *coli* O157:H7 in a sample, said sample comprising nucleic acids, said method comprising:
(a) providing a reaction mixture comprising a suitable primer pair for amplification of a polynucleotide sequence comprising SEQ ID N0:13;
(b) performing PCR amplification of said nucleic acids of said sample using the reaction mixture of step (a); and
(c) detecting the amplification of step (b), whereby a positive detection of amplification indicates the presence of *E*. *coli* O157:H7 in the sample.

Other objects and advantages will become apparent to those skilled in the art upon reference to the detailed description that hereinafter follows.

### SUMMARY OF THE SEQUENCES

SEQ ID NOs:1, 2, 3, 4, 5, 6, 7, and 8 are primer sequences for use in the detection of *E*. *coli* O157:H7.

SEQ ID NOs:9, 10, 11, and 12 are probe sequences for use in the detection of *E. coli* 0157:H7. In some embodiments, the probe is 5'-labeled with a fluorescent dye and 3'- termini is composed of a quencher dye. In some embodiments, the 3' termini are comprised of one of the primers listed above, for example SEQ ID NO:5, a suitable linker moiety, such as a spacer consisting of 6 polyethylene glycol units and a quencher dye.

SEQ ID NO:13 is an *Escherichia coli* sequence for detection.

### DETAILED DESCRIPTION

Applicants specifically incorporate the entire contents of all cited references in this disclosure. Further, when an amount, concentration, or other value or parameter is given as either a range or a list of upper values and lower values, this is to be understood as specifically disclosing all ranges formed from any pair of any upper range limit or value and any lower range limit or value, regardless of whether ranges are separately disclosed. Where a range of numerical values is recited herein, unless otherwise stated, the range is intended to include the endpoints thereof, and all integers and fractions within the range. It is not intended that the scope of the present disclosure be limited to the specific values recited when defining a range.

### Definitions

In this disclosure, a number of terms and abbreviations are used. The following definitions are provided.

As used herein, the term "about" or "approximately" means within 20%, 19%, 18%, 17%, 16%, 15%, 14%, 13%, 12%, 11%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, or 0.1%, or less of a given value or range.

The term "comprising" is intended to include embodiments encompassed by the terms "consisting essentially of" and "consisting of". Similarly, the term "consisting essentially of" is intended to include embodiments encompassed by the term "consisting of".

"Polymerase chain reaction" is abbreviated PCR.

The term "isolated" refers to materials, such as nucleic acid molecules and/or proteins, which are substantially free or otherwise removed from components that normally accompany or interact with the materials in a naturally occurring environment. Isolated polynucleotides may be purified from a host cell in which they naturally occur. Conventional nucleic acid purification methods known to skilled artisans may be used to obtain isolated polynucleotides. The term also embraces recombinant polynucleotides and chemically synthesized polynucleotides.

The terms "polynucleotide", "polynucleotide sequence", "nucleic acid sequence", and "nucleic acid fragment" are used interchangeably herein. These terms encompass nucleotide sequences and the like. A polynucleotide may be a polymer of RNA or DNA that is single- or double-stranded, that optionally contains synthetic, non-natural, or altered nucleotide bases. A polynucleotide may also consist of nucleotide sequences joined at the 3' end of one nucleotide sequence to the 5' end of another nucleotide sequence by a linker such as a 3 or 6 carbon (propandiol or hexandiol, respectively) moiety, or a linker arm of either 3 or 6 polyethylene glycol subunits (triethylene glycol or hexaethylene glycol, respectively). Any suitable linkers or spacers that are known in the art will work for this application. A polynucleotide in the form of a polymer of DNA may be comprised of one or more strands of cDNA, genomic DNA, synthetic DNA, or mixtures thereof.

The term "amplification product" refers to nucleic acid fragments produced during a primer-directed amplification reaction. Typical methods of primer-directed amplification include polymerase chain reaction (PCR), ligase chain reaction (LCR), or strand displacement amplification (SDA). If PCR methodology is selected, the replication composition may comprise the components for nucleic acid replication, for example: nucleotide triphosphates, two (or more) primers with appropriate sequences, thermostable polymerase, buffers, solutes, and proteins. These reagents and details describing procedures for their use in amplifying nucleic acids are provided in, e.g., U.S. Patent No. 4,683,202 (1987, Mullis et al.) and U.S. Patent No. 4,683,195 (1986, Mullis et al.). If LCR methodology is selected, then the nucleic acid replication compositions may comprise, for example: a thermostable ligase (*e.g.*, *Thermus aquaticus* ligase), two sets of adjacent oligonucleotides (wherein one member of each set is complementary to each of the target strands), Tris-HCl buffer, KCI, EDTA, NAD, dithiothreitol, and salmon sperm DNA. See, for example, Tabor et al., Proc. Natl. Acad. Sci. U.S.A. 82:1074-1078 (1985).

The term "primer" refers to an oligonucleotide (synthetic or occurring naturally) that is capable of acting as a point of initiation of nucleic acid synthesis or replication along a complementary strand when placed under conditions in which synthesis of a complementary strand is catalyzed by a polymerase. A primer can further contain a detectable label, for example a 5' end label.

The term "probe" refers to an oligonucleotide (synthetic or occurring naturally) that is complementary (though not necessarily fully complementary) to a polynucleotide of interest and forms a duplexed structure by hybridization with at least one strand of the polynucleotide of interest. A probe or primer-probe complex can further contain a detectable label.

A probe can either be an independent entity or complexed with or otherwise attached to a primer, such as where a probe is connected via its 3' terminus to a primer's 5' terminus through a linker, which may be a nucleotide or non-nucleotide linker and which may be a non-amplifiable linker, such as a hexaethylene glycol (HEG) or 18-carbon linker. In such a case, this would be termed a "primer-probe complex." One example of such a primer-probe complex can be found in U.S. Patent No. 6,326,145, which are frequently referred to as "Scorpion^{®} probes" or "Scorpion^{®} primers."

As used herein, the terms "label" and "detectable label" refer to a molecule capable of detection, including, but not limited to, radioactive isotopes, fluorescers, chemiluminescers, enzymes, enzyme substrates, enzyme cofactors, enzyme inhibitors, chromophores, dyes, metal ions, metal sols, semiconductor nanocrystals, ligands (e.g., biotin, avidin, streptavidin, or haptens), and the like. A detectable label can also include a combination of a reporter and a quencher.

The term "reporter" refers to a substance or a portion thereof which is capable of exhibiting a detectable signal, which signal can be suppressed by a quencher. The detectable signal of the reporter is, e.g., fluorescence in the detectable range. The term "quencher" refers to a substance or portion thereof which is capable of suppressing, reducing, inhibiting, etc., the detectable signal produced by the reporter.

As used herein, the terms "quenching" and "fluorescence energy transfer" refer to the process whereby, when a reporter and a quencher are in close proximity, and the reporter is excited by an energy source, a substantial portion of the energy of the excited state non-radioactively transfers to the quencher where it either dissipates non-radioactively or is emitted at a different emission wavelength than that of the reporter.

In some embodiments, the reporter may be selected from fluorescent organic dyes modified with a suitable linking group for attachment to the oligonucleotide, such as to the terminal 3' carbon or terminal 5' carbon. The quencher may also be selected from organic dyes, which may or may not be fluorescent, depending on the embodiment. Generally, whether the quencher is fluorescent or simply releases the transferred energy from the reporter by non-radiative decay, the absorption band of the quencher should at least substantially overlap the fluorescent emission band of the reporter to optimize the quenching. Non-fluorescent quenchers or dark quenchers typically function by absorbing energy from excited reporters, but do not release the energy radiatively.

Selection of appropriate reporter-quencher pairs for particular probes may be undertaken in accordance with known techniques. Fluorescent and dark quenchers and their relevant optical properties from which exemplary reporter-quencher pairs may be selected are listed and described, for example, in Berlman, Handbook of Fluorescence Spectra of Aromatic Molecules, 2nd ed., Academic Press, New York, 1971. Examples of modifying reporters and quenchers for covalent attachment via common reactive groups that can be added to an oligonucleotide in the present disclosure may be found, for example, in Haugland, Handbook of Fluorescent Probes and Research Chemicals, Molecular Probes of Eugene, Oreg., 1992.

In some embodiments, reporter-quencher pairs may be selected from xanthene dyes including fluoresceins and rhodamine dyes. Many suitable forms of these compounds are available commercially with substituents on the phenyl groups, which can be used as the site for bonding or as the bonding functionality for attachment to an oligonucleotide. In some embodiments, fluorescent compounds for use as reporters are the naphthylamines, having an amino group in the alpha or beta position. Included among such naphthylamino compounds are 1-dimethylaminonaphthyl-5 sulfonate, 1-anilino-8-naphthalene sulfonate and 2-p-touidinyl-6-naphthalene sulfonate. Other dyes include 3-phenyl-7-isocyanatocoumarin; acridines such as 9-isothiocyanatoacridine; N-(p-(2-benzoxazolyl)phenyl)maleimide; benzoxadiazoles; stilbenes; pyrenes and the like.

In some embodiments, the reporters and quenchers are selected from fluorescein and rhodamine dyes. These dyes and appropriate linking methodologies for attachment to oligonucleotides are well known in the art.

Suitable examples of quenchers may be selected from 6-carboxy-tetramethylrhodamine, 4-(4-dimethylaminophenylazo) benzoic acid (DABYL), tetramethylrhodamine (TAMRA), BHQ-0^{™}, BHQ-1^{™}, BHQ-2^{™}, and BHQ-3^{™}, each of which are available from LGC Biosearch Technologies, Inc. of Novato, Calif., QSY-7^{™}, QSY-9^{™}, QSY-21^{™} and QSY-35^{™}, each of which are available from ThermoFisher Scientific (Waltham, MA), and the like.

Suitable examples of reporters may be selected from dyes such as SYBR^{®} green, 5-carboxyfluorescein (5-FAM^{™} available from Applied Biosystems of Foster City, Calif.), 6-carboxyfluorescein (6-FAM), tetrachloro-6-carboxyfluorescein (TET), 2,7-dimethoxy-4,5-dichloro-6-carboxyfluorescein, hexachloro-6-carboxyfluorescein (HEX), 6-carboxy-2',4,7,7'-tetrachlorofluorescein (6-TET^{™} available from Applied Biosystems), carboxy-X-rhodamine (ROX), 6-carboxy-4',5'-dichloro-2',7'-dimethoxyfluorescein (6-JOE^{™} available from Applied Biosystems), VIC^{™} dye products available from ThermoFisher Scientific, NED^{™} dye products available from available from Applied Biosystems, Cal Fluor^{®} dye products (such as, e.g., Cal Fluor^{®} Gold 540, Orange 560, Red 590, Red 610, Red 635) available from LGC Biosearch Technologies, Quasar dye products (such as, e.g., Quasar 570, 670, 705) available from LGC Biosearch Technologies, and the like.

One example of a probe which contains a reporter and a quencher is a probe that is to be used in a 5'-exonuclease assay, such as the Taqman^{®} real-time PCR technique. In this context, the oligonucleotide probe will have a sufficient number of phosphodiester linkages adjacent to its 5' end so that the 5' to 3' nuclease activity employed can efficiently degrade the bound probe to separate the reporters and quenchers.

Another example of a probe which contains a reporter and a quencher is a Scorpion^{®} probe in either a unimolecular or bimolecular conformation. In a unimolecular Scorpion^{®}, the probe portion of the primer-probe complex is flanked by self-complementary regions which allow the probe to form into a stem-loop structure when the probe is unbound from its target DNA. Further, in a unimolecular Scorpion^{®}, a reporter is typically attached at or near one of the self-complementary regions, such as at the 5' terminus of the Scorpion^{®} probe, and a quencher is attached at or near the other self-complementary region, such as at the 3' end of the complementary sequence and adjacent to the non-amplifiable linker, such that the quencher is in sufficiently close proximity to the reporter to cause quenching when the probe is in its stem-loop conformation. In a bimolecular Scorpion^{®}, self-complementary flanking regions are not typically employed, but rather a separate "blocking oligonucleotide" is employed in conjunction with the Scorpion^{®} probe. This blocking oligonucleotide is capable of hybridizing to the probe region of the Scorpion^{®} probe when the probe is unbound from its target DNA. Further, in a bimolecular Scorpion^{®}, the reporter is typically attached to the probe region of the Scorpion^{®} probe, such as at the 5' terminus of the Scorpion^{®} probe, while the quencher is attached to the blocking oligonucleotide, such as at the 3' terminus of the blocking oligonucleotide, such that the quencher is in sufficiently close proximity to the reporter to cause quenching when the probe is unbound from its target DNA and is instead hybridized to the blocking oligonucleotide.

Yet another example of a probe which contains a reporter and quencher is a Molecular Beacon type probe, which contains a probe region flanked by self-complementary regions that allow the probe to form a stem-loop structure when unbound from the probe's target sequence. Such probes typically have a reporter attached at or near one terminus and a quencher attached at or near the other terminus such that the quencher is in sufficiently close proximity to the reporter to cause quenching when the probe is in its unbound, and thus stem-loop, form.

The term "replication inhibitor moiety" refers to any atom, molecule or chemical group that is attached to the 3' terminal hydroxyl group of an oligonucleotide that will block the initiation of chain extension for replication of a nucleic acid strand. Examples include, but are not limited to: 3'-deoxynucleotides (e.g., cordycepin), dideoxynucleotides, phosphate, ligands (e.g., biotin and dinitrophenol), reporter molecules (e.g., fluorescein and rhodamine), carbon chains (e.g., propanol), a mismatched nucleotide or polynucleotide, or peptide nucleic acid units. The term "non-participatory" refers to the lack of participation of a probe or primer in a reaction for the amplification of a nucleic acid molecule. Specifically, a non-participatory probe or primer is one that will not serve as a substrate for, or be extended by, a DNA or RNA polymerase. A "non-participatory probe" is inherently incapable of being chain extended by a polymerase. It may or may not have a replication inhibitor moiety.

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength. Hybridization and washing conditions are well known and exemplified, for example, in Sambrook, J., Fritsch, E. F. and Maniatis, T., Molecular Cloning: A Laboratory Manual, 2nd ed., Cold Spring Harbor Laboratory: Cold Spring Harbor, NY (1989), particularly Chapter 11 and Table 11.1 therein. The conditions of temperature and ionic strength determine the "stringency" of the hybridization. Examples of salt concentration ranges and temperature ranges for different hybridization conditions are as follows: high stringency, approximately 0.01 M to approximately 0.05 M salt, hybridization temperature 5 °C to 10 °C below Tm; moderate stringency, approximately 0.16 M to approximately 0.33 M salt, hybridization temperature 20 °C to 29 °C below Tm; low stringency, approximately 0.33 M to approximately 0.82 M salt, hybridization temperature 40 °C to 48 °C below T*m*. For preliminary screening for homologous nucleic acids, low stringency hybridization conditions, corresponding to a Tm of 55°C, can be used, e.g., 5X SSC, 0.1% SDS, 0.25% milk, and no formamide; or 30% formamide, 5X SSC, 0.5% SDS. Moderate stringency hybridization conditions correspond to a higher T*m*, e.g., 40% formamide, with 5X or 6X SSC. Hybridization requires that the two nucleic acids contain complementary sequences, although, depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of T*m* for hybrids of nucleic acids having those sequences. The relative stability (corresponding to higher T*m*) of nucleic acid hybridizations decreases in the following order: RNA:RNA, DNA:RNA, DNA:DNA. For hybrids of greater than 100 nucleotides in length, equations for calculating T*m* have been derived (see Sambrook et al., *supra*, 9.50-9.51). Algorithm prediction tools to estimate T*m* are also widely available. For hybridizations with shorter nucleic acids, i.e., oligonucleotides, the position of mismatches becomes more important, and the length of the oligonucleotide determines its specificity (see Sambrook et al., *supra*, 11.7-11.8). In some embodiments, the length for a hybridizable nucleic acid is at least about 10 nucleotides, at least about 11 nucleotides, at least about 12 nucleotides, at least about 13 nucleotides, at least about 14 nucleotides, at least about 15 nucleotides, at least about 16 nucleotides, at least about 17 nucleotides, at least about 18 nucleotides, at least about 19 nucleotides, at least about 20 nucleotides, at least about 21 nucleotides, at least about 22 nucleotides, at least about 23 nucleotides, at least about 24 nucleotides, at least about 25 nucleotides, at least about 26 nucleotides, at least about 27 nucleotides, at least about 28 nucleotides, at least about 29 nucleotides, or, at least about 30 nucleotides or more. Furthermore, the skilled artisan will recognize that the temperature and wash solution salt concentration may be adjusted as necessary according to factors such as length of the probe.

Standard recombinant DNA and molecular cloning techniques used here are well known in the art and are described by, e.g., Sambrook et al. (supra); and by Ausubel, F. M. et al., Current Protocols in Molecular Biology, published by Greene Publishing Assoc. and Wiley-Interscience (1987).

### Genome Detection Regions

Applicants have solved the stated problem through a method that uses a pair of phage protein sequences the amplification of which is only detectable in *E. coli* 0157:H7. The use of this target eliminates the potential for false positive results in mixed cultures of *E*. *coli.*

The present detection method finds utility in detection of *E*. *coli* 0157:H7 in any type of sample, for example in appropriate samples for food testing, environmental testing, or human or animal diagnostic testing. While examples of suitable methods for detecting these regions are included herein, it is to be understood that use of the presently disclosed sequences is not limited to the methods described herein. Rather any suitable method can be employed to detect these DNA regions and subsequently the *E. coli* itself.

### Oligonucleotides

Oligonucleotides of the instant disclosure are set forth in SEQ ID NOs: 1-12.

Oligonucleotides of the instant disclosure may be used as primers for PCR amplification. Exemplary primer pairs and their corresponding probes are shown in Table 1.

**TABLE 1**

| **5' (Forward) Primer** | **3' (Reverse) Primer** | **Probe** |
|---|---|---|
| SEQ ID NO:1 | SEQ ID NO:2 | SEQ ID NO:9 |
| SEQ ID NO:3 | SEQ ID NO:4 | SEQ ID NO:10 |
| SEQ ID NO:5 | SEQ ID NO:6 | SEQ ID NO:11 |
| SEQ ID NO:7 | SEQ ID NO:8 | SEQ ID NO:12 |

These oligonucleotide primers may also be useful for other nucleic acid amplification methods such as the ligase chain reaction (LCR) (Backman et al., 1989, EP 0 320 308; Carrino et al., 1995, J. Microbiol. Methods 23: 3-20); nucleic acid sequence-based amplification (NASBA) (Carrino *et al.*, 1995, *supra*); and self-sustained sequence replication (3SR) and 'Q replicase amplification' (Pfeffer et al., 1995 Veterinary Res. Comm. 19: 375-407).

In some embodiments, the oligonucleotide primers can also contain a detectable label, for example a 5' end label.

In addition, oligonucleotides also may be used in some embodiments as hybridization probes. In some embodiments, hybridization probes can be SEQ ID NO:9, SEQ ID NO:10, SEQ ID NO:11, or SEQ ID NO:12. Hybridization using DNA probes has been frequently used for the detection of pathogens in food, clinical and environmental samples, and the methodologies are generally known to one skilled in the art. It is generally recognized that the degree of sensitivity and specificity of probe hybridization is lower than that achieved through the previously described amplification techniques. The nucleic acid probes can also possess a detectable label, such as a reporter-quencher combination as are employed in 5'-exonuclease detection assays, such as the Taqman^{®} assay or in Scorpion^{®} probe assays.

The 3' terminal nucleotide of the nucleic acid probe may be rendered incapable of extension by a nucleic acid polymerase in some embodiments. Such blocking may be carried out, for example by the attachment of a replication inhibitor moiety, such as a reporter or quencher, to the terminal 3' carbon of the nucleic acid probe by a linking moiety, or by making the 3'-terminal nucleotide a dideoxynucleotide. Alternatively, the 3' end of the nucleic acid probe may be rendered impervious to the 3' to 5' extension activity of a polymerase by incorporating one or more modified internucleotide linkages onto the 3' end of the oligonucleotide. Minimally, the 3' terminal internucleotide linkage must be modified; however, additional internucleotide linkages may be modified. Internucleotide modifications which prevent elongation from the 3' end of the nucleic acid probe and/or which block the 3' to 5' exonuclease activity of the DNA polymerase during PCR may include phosphorothioate linkages, methylphosphonate linkages, boranophosphate linkages, and other similar polymerase-resistant internucleotide linkages. An alternative method to block 3' extension of the probe is to form an adduct at the 3' end of the probe using mitomycin C or other like antitumor antibiotics such as described in Basu et al., Biochemistry 32:4708-4718, 1993. Thus, the precise mechanism by which the 3' end of the nucleic acid probe is protected from cleavage is not essential so long as the quencher is not cleaved from the nucleic acid probe.

A nucleic acid probe sequence can also optionally be employed with the primer sequence pairs of the present disclosure in an amplification-based detection technique, such as in the 3'-exonuclease assay. Exemplary primer/probe combinations are indicated in Table 1.

### Assay Methods

Detection of the presence of *E. coli* O157:H7 itself may be accomplished in any suitable manner. Exemplary methods are primer-directed amplification methods and nucleic acid hybridization methods. These methods may be used to detect E. *coli* 0157:H7 in a sample that is either a complex matrix or a purified culture, e.g., from an animal, environmental, or food source suspected of contamination.

In some embodiments, the assay method comprises (1) culturing a complex sample mixture in a non-selective growth media to resuscitate the target bacteria, (2) releasing total target bacterial DNA, and (3) subjecting the total DNA to an amplification protocol with a primer pair, such as a primer pair disclosed herein, and optionally with a nucleic acid probe comprising a detectable label.

### Primer-Directed Amplification Assay Methods

A variety of primer-directed nucleic acid amplification methods are known in the art which can be employed, including thermal cycling methods (e.g., PCR, RT-PCR, and LCR), as well as isothermal methods and strand displacement amplification (SDA). In some embodiments, the method is PCR. In some embodiments, the primer pairs listed in Table 1 may be used as primers for use in primer-directed nucleic acid amplification for the detection of SEQ ID NOs: 1-3 and subsequently detection and identification of *E. coli* 0157:H7.

### Sample Preparation:

The oligonucleotides and methods disclosed herein may be used directly with any suitable clinical or environmental samples, without any need for sample preparation. In order to achieve higher sensitivity, and in situations where time is not a limiting factor, the samples can be pre-treated and then pre-amplification enrichment is performed.

The minimum industry standard for the detection of food-borne bacterial pathogens is a method that will reliably detect the presence of one pathogen cell in 25 g of food matrix as described in Andrews et al., 1984, "Food Sample and Preparation of Sample Homogenate", Chapter 1 in Bacteriological Analytical Manual, 8th Edition, Revision A, Association of Official Analytical Chemists, Arlington, VA. In order to satisfy this stringent criterion, enrichment methods and media have been developed to enhance the growth of the target pathogen cell in order to facilitate its detection by biochemical, immunological or nucleic acid hybridization means. Typical enrichment procedures employ media that will enhance the growth and health of the target bacteria and also inhibit the growth of any background or non-target microorganisms present. For example, the USDA has set forth a protocol for enrichment of samples of ground beef to be tested for pathogenic *E*. *coli* (FDA Bacteriological Analytical Manual. (1998) published and distributed by the Association of Analytical Chemists, Suite 400, 2200 Wilson Blvd, Arlington, VA 22201-3301; available on the U.S. Food and Drug Administration website, https://www.fda.gov/food/foodscienceresearch/ laboratorymethods/ucm2006949.htm).

Selective media have been developed for a variety of bacterial pathogens and one of skill in the art will know to select a medium appropriate for the particular organism to be enriched, e.g. *E. coli* O157:H7. A general discussion and recipes of non-selective media are described in the FDA Bacteriological Analytical Manual (supra).

After selective growth, a sample of the complex mixtures is removed for further analysis. This sampling procedure may be accomplished by a variety of means well known to those skilled in the art. In some embodiments, 5 µl of the enrichment culture is removed and added to 200 µl of lysis solution containing protease. The lysis solution is heated at 37°C for 20 min followed by protease inactivation at 95°C for 10 min as described in the BAX^{®} System User's Guide, Hygiena Qualicon, Inc., Wilmington, DE (available on the Hygiena website, https://www.hygiena.com/index.php?option= com_docman&view=document&alias=945-ins2025-e-coli-o157h7-mp-rev-03&category_slug=qualicon-instructions&Itemid=1134).

### PCR Assay Methods:

In some embodiments, a method for detecting the presence of *E*. *coli* 0157:H7 in a sample comprises (a) performing PCR amplification using primer pairs listed in Table 1 to produce a PCR amplification result; and (b) detecting the amplification, whereby a positive detection of the amplification indicates the presence of *E. coli* 0157:H7 in the sample.

In some embodiments, prior to performing PCR amplification, a step of preparing the sample may be carried out. The preparing step may comprise at least one of the following processes: (1) bacterial enrichment, (2) separation of bacterial cells from the sample, (3) cell lysis, and (4) total DNA extraction.

### Amplification Conditions:

A skilled person will understand that any generally acceptable PCR condition may be used for successfully detecting *E. coli* O157:H7 bacteria using the oligonucleotides of the instant disclosure, and depending on the sample to be tested and other laboratory conditions, routine optimization for the PCR conditions may be necessary to achieve optimal sensitivity and specificity. Optimally, PCR amplification results may be achieved from all of the intended specific targets while giving no PCR results for other, non-target species.

### Detection/Examination/Analysis:

Primer-directed amplification products can be analyzed using various methods. "Homogenous detection" refers to a method for the detection of amplification products where no separation (such as by gel electrophoresis) of amplification products from template or primers is necessary. Homogeneous detection is typically accomplished by measuring the level of fluorescence of the reaction mixture during or immediately following amplification. In addition, heterogeneous detection methods, which involve separation of amplification products during or prior to detection, can be employed in the present methods.

Homogenous detection may be employed to carry out "real-time" primer-directed nucleic acid amplification and detection, using primer pairs of the instant disclosure (e.g., "real-time" PCR and "real-time" RT-PCR). Exemplary "real-time" methods are set forth in U.S. Patent Nos. 6,171,785, 5,994,056, 6,326,145, 5,804,375, 5,538,848, 5,487,972, and 5,210,015.

In some embodiments, the "real-time" detection method is the 5'-exonuclease detection method, as set forth in U.S. Patent Nos. 5,804,375, 5,538,848, 5,487,972, and 5,210,015. In the 5'-exonuclease detection assay, a modified probe is employed during PCR which binds intermediate to or between the two members of the amplification primer pair. The modified probe possesses a reporter and a quencher and is designed to generate a detectable signal to indicate that it has hybridized with the target nucleic acid sequence during PCR. As long as both the reporter and the quencher are on the probe, the quencher stops the reporter from emitting a detectable signal. However, as the polymerase extends the primer during amplification, the intrinsic 5' to 3' nuclease activity of the polymerase degrades the probe, separating the reporter from the quencher, and enabling the detectable signal to be emitted. Generally, the amount of detectable signal generated during the amplification cycle is proportional to the amount of product generated in each cycle.

It is well known that the efficiency of quenching is a strong function of the proximity of the reporter and the quencher, i.e., as the two molecules get closer, the quenching efficiency increases. As quenching is strongly dependent on the physical proximity of the reporter and quencher, the reporter and the quencher are, in some embodiments, attached to the probe within a few nucleotides of one another, usually within 30, 29, 28, 27, 26, 25, 24, 23, 22, 21, 20, 19, 18, 17, 16, 15, 14, 13, 12, 11, 10, 9, 8, 7, or 6 nucleotides of one another. Typically, this separation is achieved by attaching one member of a reporter-quencher pair to the 5' end of the probe and the other member to a nucleotide about 30, 29, 28, 27, 26, 25, 24, 23, 22, 21, 20, 19, 18, 17, 16, 15, 14, 13, 12, 11, 10, 9, 8, 7, or 6 nucleotides away.

In some embodiments, amplification and detection is performed using labeled Taqman^{®} probes. SEQ ID NOs: 9 - 12 possess, in some embodiments, a CAL Fluor 610 reporter attached at the 5' terminus and a BHQ2 quencher attached at the 3' terminus.

Another "real-time" detection method is the Scorpion^{®} probe assay as set forth in U.S. Patent No. 6,326,145. In the Scorpion^{®} probe assay, PCR amplification is performed using a Scorpion^{®} probe (either unimolecular or bimolecular) as a primer-probe complex, the Scorpion^{®} probe possessing an appropriate reporter-quencher pair to allow the detectable signal of the reporter to be quenched prior to elongation of the primer. Post-elongation, the quenching effect is eliminated, and the amount of signal present is quantitated. As the amount of amplification product increases, an equivalent increase in detectable signal will be observed, thus allowing the amount of amplification product present to be determined as a function of the amount of detectable signal measured. When more than one Scorpion^{®} probe is employed in a Scorpion^{®} probe assay, such as one directed to more than one DNA region of interest (such as, e.g., one probe directed to SEQ ID NO:13 and a second probe directed to a different target region), each probe can have a different detectable label (e.g., reporter-quencher pair) attached, thus allowing each probe to be detected independently of the other probes.

Another method of homogenous detection involves the use of DNA melting curve analysis, particularly with the BAX^{®} System hardware and reagent tablets from Hygiena Qualicon Inc. (Wilmington, DE). The details of the system are given in U.S. Patent No. 6,312,930 and PCT Publication Nos. WO 97/11197 and WO 00/66777.

Melting curve analysis detects and quantifies double stranded nucleic acid molecule ("dsDNA" or "target") by monitoring the fluorescence of the target amplification product ("target amplicon") during each amplification cycle at selected time points.

As is well known to the skilled artisan, the two strands of a dsDNA separate or melt, when the temperature is higher than its melting temperature. Melting of a dsDNA molecule is a process, and under a given solution condition, melting starts at a temperature (designated T_{MS} hereinafter), and completes at another temperature (designated T_{ME} hereinafter). The familiar term, Tₘ, designates the temperature at which melting is 50% complete.

A typical PCR cycle involves a denaturing phase where the target dsDNA is melted, a primer annealing phase where the temperature optimal for the primers to bind to the now-single-stranded target, and a chain elongation phase (at a temperature T_{E}) where the temperature is optimal for DNA polymerase to function.

In a melt curve analysis, T_{MS} should be higher than T_{E}, and T_{ME} should be lower (often substantially lower) than the temperature at which the DNA polymerase is heat-inactivated. Melting characteristics are affected by the intrinsic properties of a given dsDNA molecule, such as deoxynucleotide composition and the length of the dsDNA.

Intercalating dyes will bind to double stranded DNA. The dye/dsDNA complex will fluoresce when exposed to the appropriate excitation wavelength of light, which is dye dependent, and the intensity of the fluorescence may be proportionate to concentration of the dsDNA. Methods taking advantage of the use of DNA intercalating dyes to detect and quantify dsDNA are known in the art. Many dyes are known and used in the art for these purposes. The instant methods also take advantage of such relationship.

Examples of such intercalating dyes include, but are not limited to, SYBR^{®} Green-I, ethidium bromide, propidium iodide, TOTO^{®}-1 {Quinolinium, 1-1'-[1,3-propanediylbis [(dimethyliminio)-3,1-propanediyl]]bis[4-[(3-methyl-2(3H)-benzothiazolylidene) methyl]]-, tetraiodide}, and YoPro^{®} {Quinolinium, 4-[(3-methyl-2(3H)-benzoxazolylidene)methyl]-1-[3-(trimethylammonio)-propyl]-,diiodide}. In some embodiments, a non-asymmetrical cyanide dye such as SYBR^{®} Green-I, manufactured by ThermoFisher Scientific, is the intercalating dye.

Melting curve analysis is achieved by monitoring the change in fluorescence while the temperature is increased. When the temperature reaches the T_{MS} specific for the target amplicon, the dsDNA begins to denature. When the dsDNA denatures, the intercalating dye dissociates from the DNA and fluorescence decreases. Mathematical analysis of the negative of the change of the log of fluorescence divided by the change in temperature plotted against the temperature results in the graphical peak known as a melting curve.

It should be understood that the present methods could be operated using a combination of techniques, such as by having a Scorpion^{®} probe directed to one target region and a Taqman^{®} probe directed to a second target region. It should also be understood that the methods are not limited to the above described techniques. Rather, one skilled in the art would recognize that other techniques for detecting amplification as known in the art may also be used. For example, techniques such as PCR-based quantitative sequence detection (QSD) may be performed using nucleic acid probes which, when present in the single-stranded state in solution, are configured such that the reporter and quencher are sufficiently close to substantially quench the reporter's emission. However, upon hybridization of the intact reporter-quencher nucleic acid probe with the amplified target nucleic acid sequence, the reporter and quenchers become sufficiently distant from each other. As a result, the quenching is substantially abated causing an increase in the fluorescence emission detected.

In addition to homogenous detection methods, a variety of other heterogeneous detection methods are known in the art which can be employed in the present methods, including standard non-denaturing gel electrophoresis (e.g., acrylamide or agarose), denaturing gradient gel electrophoresis, and temperature gradient gel electrophoresis. Standard non-denaturing gel electrophoresis is a simple and quick method of PCR detection but may not be suitable for all applications.

Denaturing Gradient Gel Electrophoresis (DGGE) is a separation method that detects differences in the denaturing behavior of small DNA fragments (200-700 bp). The principle of the separation is based on both fragment length and nucleotide sequence. In fragments that are the same length, a difference as little as one base pair can be detected. This is in contrast to non-denaturing gel electrophoresis, where DNA fragments are separated only by size. This limitation of non-denaturing gel electrophoresis results because the difference in charge density between DNA molecules is near neutral and plays little role in their separation. As the size of the DNA fragment increases, its velocity through the gel decreases.

DGGE is primarily used to separate DNA fragments of the same size based on their denaturing profiles and sequence. Using DGGE, two strands of a DNA molecule separate, or melt, when heat or a chemical denaturant is applied. The denaturation of a DNA duplex is influenced by two factors: 1) the hydrogen bonds formed between complimentary base pairs (since GC rich regions melt at higher denaturing conditions than regions that are AT rich); and 2) the attraction between neighboring bases of the same strand, or "stacking". Consequently, a DNA molecule may have several melting domains with each of their individual characteristic denaturing conditions determined by their nucleotide sequence. DGGE exploits the fact that otherwise identical DNA molecules having the same length and DNA sequence, with the exception of only one nucleotide within a specific denaturing domain, will denature at different temperatures or Tm. Thus, when the double-stranded (ds) DNA fragment is electrophoresed through a gradient of increasing chemical denaturant, it begins to denature and undergoes both a conformational and mobility change. The dsDNA fragment will travel faster than a denatured single-stranded (ss) DNA fragment, since the branched structure of the single-stranded moiety of the molecule becomes entangled in the gel matrix. As the denaturing environment increases, the dsDNA fragment will completely dissociate and mobility of the molecule through the gel is retarded at the denaturant concentration at which the particular low denaturing domains of the DNA strand dissociate. In practice, the electrophoresis is conducted at a constant temperature (around 60°C) and chemical denaturants are used at concentrations that will result in 100% of the DNA molecules being denatured (e.g.., 40% formamide and 7M urea). This variable denaturing gradient is created using a gradient maker, such that the composition of each DGGE gel gradually changes from 0% denaturant up to 100% denaturant. Of course, gradients containing a reduced range of denaturant (e.g., 35% to 60%) may also be poured for increased separation of DNA.

The principle used in DGGE can also be applied to a second method that uses a temperature gradient instead of a chemical denaturant gradient. This method is known as Temperature Gradient Gel Electrophoresis (TGGE). This method makes use of a temperature gradient to induce the conformational change of dsDNA to ssDNA to separate fragments of equal size with different sequences. As in DGGE, DNA fragments with different nucleotide sequences will become immobile at different positions in the gel. Variations in primer design can be used to advantage in increasing the usefulness of DGGE for characterization and identification of the PCR products. These methods and principles of using primer design variations are described in PCR Technology Principles and Applications, Henry A. Erlich Ed., M. Stockton Press, NY, pages 71 to 88 (1988).

### Instrumentation:

When homogenous detection is employed, the level of fluorescence is, in some embodiments, measured using a laser fluorometer such as, for example, an ABI Prism Model 7500 Fast Sequence Detector. However, similar detection systems for measuring the level of fluorescence in a sample can be used in the methods disclosed herein.

### Reagents and Kits:

Any suitable nucleic acid replication composition ("replication composition") in any format can be used. A typical replication composition for PCR amplification may comprise, for example, dATP, dCTP, dGTP, dTTP, target specific primers and a suitable polymerase.

If the replication composition is in liquid form, suitable buffers known in the art may be used (Sambrook, J. et al., supra).

Alternatively, if the replication composition is contained in a tablet form, then typical tabletization reagents may be included such as stabilizers and binding agents. Exemplary tabletization technology is set forth in U.S. Patent Nos. 4,762,857 and 4,678,812.

In some embodiments, the replication composition can comprise (a) at least one primer pair selected from Table 1, and (b) a thermostable DNA polymerase. In some embodiments, the replication composition can comprise (a) at least two primer pairs selected from Table 1, each directed toward a different target DNA region; and (b) a thermostable DNA polymerase. In some embodiments, at least one primer pair is directed to SEQ ID NO:13.

In some embodiments, the replication composition can comprise (a) at least two primer pairs and any corresponding probe or blocking oligonucleotide selected from Table 1, wherein each nucleic acid probe or primer-probe complex employed comprises a detectable label; and (b) a thermostable DNA polymerase. The detectable label can comprise, in some embodiments, a reporter capable of emitting a detectable signal and a quencher capable of substantially quenching the reporter and preventing the emission of the detectable signal when the reporter and quencher are in sufficiently close proximity to one another.

Kits disclosed herein can comprise any one of the above replication compositions. Tablets disclosed herein can comprise any one of the above replication compositions. In some embodiments, a kit can comprise tablet comprising any one of the above replication compositions.

In some instances, an internal positive control can be included in the reaction. The internal positive control can include control template nucleic acids (e.g. DNA or RNA), control primers, and control nucleic acid probe. The advantages of an internal positive control contained within a PCR reaction have been previously described (U.S. Patent No. 6,312,930 and PCT Application No. WO 97/11197), and include: (i) the control may be amplified using a single primer; (ii) the amount of the control amplification product is independent of any target DNA or RNA contained in the sample; (iii) the control DNA can be tableted with other amplification reagents for ease of use and high degree of reproducibility in both manual and automated test procedures; (iv) the control can be used with homogeneous detection, i.e., without separation of product DNA from reactants; and (v) the internal control has a melting profile that is distinct from other potential amplification products in the reaction and/or a detectable label on the control nucleic acid that is distinct from the detectable label on the nucleic acid probe directed to the target.

Control DNA will be of appropriate size and base composition to permit amplification in a primer-directed amplification reaction. The control template DNA sequence may be obtained from the *E. coli* genome, or from another source, but must be reproducibly amplified under the same conditions that permit the amplification of the target amplification product.

Control sequences can include, for example, those found in SV40. The concentration range of SV40, when used, can be for example 10¹, 10², 10³, 10⁴, 10⁵, 10⁶, or 10⁷ copies per PCR reaction.

The control reaction is useful to validate the amplification reaction. Amplification of the control DNA occurs within the same reaction tube as the sample that is being tested, and therefore indicates a successful amplification reaction when samples are target negative, i.e. no target amplification product is produced. In order to achieve significant validation of the amplification reaction, a suitable number of copies of the control DNA template must be included in each amplification reaction.

In some instances, it may be useful to include an additional negative control replication composition. The negative control replication composition will contain the same reagents as the replication composition but without the polymerase. The primary function of such a control is to monitor spurious background fluorescence in a homogeneous format when the method employs a fluorescent means of detection.

Replication compositions may be modified depending on whether they are designed to be used to amplify target DNA or the control DNA. Replication compositions that will amplify the target DNA (test replication compositions) may include (i) a polymerase (generally thermostable), (ii) a primer pair capable of hybridizing to the target DNA and (iii) necessary buffers for the amplification reaction to proceed. Replication compositions that will amplify the control DNA (positive control, or positive replication composition) may include (i) a polymerase (generally thermostable) (ii) the control DNA; (iii) at least one primer capable of hybridizing to the control DNA; and (iv) necessary buffers for the amplification reaction to proceed. In addition, the replication composition for either target DNA or control DNA amplification can contain a nucleic acid probe, in some embodiments possessing a detectable label.

### Nucleic Acid Hybridization Methods

In addition to primer-directed amplification assay methods, nucleic acid hybridization assay methods can be employed for detection of *E*. *coli* O157:H7. The basic components of a nucleic acid hybridization test include a probe, a sample suspected of containing *E. coli* O157:H7, and a specific hybridization method. Typically, the probe length can vary from as few as 5 bases to the full length of the *E*. *coli* diagnostic sequence and will depend upon the specific test to be done. Only part of the probe molecule need be complementary to the nucleic acid sequence to be detected. In addition, the complementarity between the probe and the target sequence need not be perfect. Hybridization does occur between imperfectly complementary molecules with the result that a certain fraction of the bases in the hybridized region are not paired with the proper complementary base.

Probes particularly useful in nucleic acid hybridization methods are any of SEQ ID NOs:1-12, or sequences derived therefrom.

The sample may or may not contain *E. coli* 0157:H7. The sample may take a variety of forms; however, the sample will generally be extracted from an animal, environmental or food source suspected of contamination. The DNA may be detected directly but, in some embodiments, the sample nucleic acid must be made available to contact the probe before any hybridization of probe and target molecule can occur. Thus the organism's DNA is in some embodiments free from the cell and placed under the proper conditions before hybridization can occur. Methods of in-solution hybridization necessitate the purification of the DNA in order to be able to obtain hybridization of the sample DNA with the probe. This has meant that utilization of the in-solution method for detection of target sequences in a sample requires that the nucleic acids of the sample must first be purified to eliminate protein, lipids, and other cell components, and then contacted with the probe under hybridization conditions. Methods for the purification of the sample nucleic acid are common and well known in the art (Sambrook et al., *supra*)*.*

In some embodiments, hybridization assays may be conducted directly on cell lysates, without the need to extract the nucleic acids. This eliminates several steps from the sample-handling process and speeds up the assay. To perform such assays on crude cell lysates, a chaotropic agent is typically added to the cell lysates prepared as described above. The chaotropic agent stabilizes nucleic acids by inhibiting nuclease activity. Furthermore, the chaotropic agent allows sensitive and stringent hybridization of short oligonucleotide probes to DNA at room temperature (Van Ness and Chen, Nucl. Acids Res. 19:5143-5151 (1991)). Suitable chaotropic agents include guanidinium chloride, guanidinium thiocyanate, sodium thiocyanate, lithium tetrachloroacetate, sodium perchlorate, rubidium tetrachloroacetate, potassium iodide, and cesium trifluoroacetate, among others. Typically, the chaotropic agent will be present at a final concentration of about 3M. If desired, one can add formamide to the hybridization mixture, typically 30-50% (v/v).

Alternatively, one can purify the sample nucleic acids prior to probe hybridization. A variety of methods are known to one of skill in the art (e.g., phenolchloroform extraction, IsoQuick extraction (MicroProbe Corp., Bothell, WA), and others). Pre-hybridization purification is particularly useful for standard filter hybridization assays. Furthermore, purification facilitates measures to increase the assay sensitivity by incorporating *in vitro* RNA amplification methods such as self-sustained sequence replication (see for example Fahy et al., In PCR Methods and Applications, Cold Spring Harbor Laboratory: Cold Spring Harbor, NY (1991), pp. 25-33) or reverse transcriptase PCR (Kawasaki, In PCR Protocols: A Guide to Methods and Applications, M. A. Innis et al., Eds., (1990), pp. 21-27).

Once the DNA is released, it can be detected by any of a variety of methods. However, the most useful embodiments have at least some characteristics of speed, convenience, sensitivity, and specificity.

Hybridization methods are well known in the art. Typically the probe and sample must be mixed under conditions which will permit nucleic acid hybridization. This involves contacting the probe and sample in the presence of an inorganic or organic salt under the proper concentration and temperature conditions. The probe and sample nucleic acids must be in contact for a long enough time that any possible hybridization between the probe and sample nucleic acid may occur. The concentration of probe or target in the mixture will determine the time necessary for hybridization to occur. The higher the probe or target concentration, the shorter the hybridization incubation time needed.

Various hybridization solutions can be employed. Typically, these comprise from about 20 to 60% volume, e.g., about 20%, about 25%, about 30%, about 35%, about 40%, about 45%, about 50%, about 55%, or about 60%, of a polar organic solvent. A common hybridization solution employs about 30-50% (e.g., about 30%, about 35%, about 40%, about 45%, or about 50%) v/v formamide, about 0.15 M to 1 M (e.g., about 0.15 M, about 0.20 M, about 0.25 M, about 0.30 M, about 0.35 M, about 0.35 M, about 0.40 M, about 0.45 M, about 0.50 M, about 0.55 M, about 0.60 M, about 0.65 M, about 0.70 M, about 0.75 M, about 0.80 M, about 0.85 M, about 0.90 M, about 0.95 M, or about 1 M) sodium chloride, about 0.05 M to 0.1 M (e.g., about 0.05 M, about 0.10 M, about 0.15 M, about 0.20 M, about 0.25 M, about 0.30 M, about 0.35 M, about 0.35 M, about 0.40 M, about 0.45 M, about 0.50 M, about 0.55 M, about 0.60 M, about 0.65 M, about 0.70 M, about 0.75 M, about 0.80 M, about 0.85 M, about 0.90 M, about 0.95 M, or about 1 M) buffers, such as sodium citrate, Tris-HCl, PIPES or HEPES (pH range about 6-9 (e.g., about pH 6.0, about pH 6.5, about pH 7.0, about pH 7.5, about pH 8.0, about pH 8.5, or about pH 9.0), about 0.05% to 0.2% (e.g., about 0.05%, about 0.10%, about 0.15%, or about 0.20%) detergent, such as sodium dodecylsulfate, or between 0.5 mM - 20 mM (e.g., about 0.5 mM, about 1.0 mM, about 1.5 mM, about 2.0 mM, about 2.5 mM, about 3.0 mM, about 3.5 mM, about 4.0 mM, about 4.5 mM, about 5.0 mM, about 5.5 mM, about 6.0 mM, about 6.5 mM, about 7.0 mM, about 7.5 mM, about 8.0 mM, about 8.5 mM, about 9.0 mM, about 9.5 mM, about 10.0 mM, about 10.5 mM, about 11.0 mM, about 11.5 mM, about 12.0 mM, about 12.5 mM, about 13.0 mM, about 13.5 mM, about 14.0 mM, about 14.5 mM, about 15.0 mM, about 15.5 mM, about 16.0 mM, about 16.5 mM, about 17.0 mM, about 17.5 mM, about 18.0 mM, about 18.5 mM, about 19.0 mM, about 19.5 mM, or about 20.0 mM) EDTA, FICOLL (Pharmacia Inc.) (about 300-500 kilodaltons), polyvinylpyrrolidone (about 250-500 kdal), and serum albumin. Also included in the typical hybridization solution will be unlabeled carrier nucleic acids from about 0.1 mg/ml to 5 mg/ml (e.g., about 0.1 mg/ml, about 0.2 mg/ml, about 0.3 mg/ml, about 0.4 mg/ml, about 0.5 mg/ml, about 1.0 mg/ml, about 1.5 mg/ml, about 2.0 mg/ml, about 2.5 mg/ml, about 3.0 mg/ml, about 3.5 mg/ml, about 4.0 mg/ml, about 4.5 mg/ml, or about 5.0 mg/ml), fragmented nucleic DNA (e.g., calf thymus or salmon sperm DNA, or yeast RNA), and optionally from about 0.5% to 2% (e.g., about 0.05%, about 0.10%, about 0.15%, about 0.20%, about 0.25%, about 0.30%, about 0.35%, about 0.40%, about 0.45%, about 0.50%, about 0.55%, about 0.60%, about 0.65%, about 0.70%, about 0.75%, about 0.80%, about 0.85%, about 0.90%, about 0.95%, about 1.00%, about 1.05%, about 1.10%, about 1.15%, about 1.20%, about 1.25%, about 1.30%, about 1.35%, about 1.40%, about 1.45%, about 1.50%, about 1.55%, about 1.60%, about 1.65%, about 1.70%, about 1.75%, about 1.80%, about 1.85%, about 1.90%, about 1.95%, or about 2.00%,) wt/vol glycine. Other additives may also be included, such as volume exclusion agents which include a variety of polar water-soluble or swellable agents (e.g., polyethylene glycol), anionic polymers (e.g., polyacrylate or polymethylacrylate), and anionic saccharidic polymers (e.g., dextran sulfate).

Nucleic acid hybridization is adaptable to a variety of assay formats. One of the most suitable is the sandwich assay format. The sandwich assay is particularly adaptable to hybridization under non-denaturing conditions. A primary component of a sandwich-type assay is a solid support. The solid support has adsorbed to it or covalently coupled to it immobilized nucleic acid probe that is unlabeled and complementary to one portion of the DNA sequence.

The sandwich assay may be encompassed in an assay kit. This kit would include a first component for the collection of samples suspected of contamination and buffers for the disbursement and lysis of the sample. A second component would include media in either dry or liquid form for the hybridization of target and probe polynucleotides, as well as for the removal of undesirable and nonduplexed forms by washing. A third component includes a solid support (dipstick) upon which is fixed (or to which is conjugated) unlabeled nucleic acid probe(s) that is (are) complementary to the target sequences. A fourth component would contain labeled probe that is complementary to a second and different region of the same DNA strand to which the immobilized, unlabeled nucleic acid probe of the third component is hybridized.

In some embodiments, SEQ ID NOs: 1-12 or derivations thereof may be used as 3' blocked detection probes in either a homogeneous or heterogeneous assay format. For example, a probe generated from these sequences may be 3' blocked or non-participatory and will not be extended by, or participate in, a nucleic acid amplification reaction. Additionally, the probe, in some embodiments, incorporates a label that can serve as a reactive ligand that acts as a point of attachment for the immobilization of the probe/analyte hybrid or as a reporter to produce detectable signal. Accordingly, genomic or cDNA isolated from a sample suspected of *E*. *coli* contamination is amplified by standard primer-directed amplification protocols in the presence of an excess of the 3' blocked detection probe to produce amplification products. Because the probe is 3' blocked, it does not participate or interfere with the amplification of the target. After the final amplification cycle, the detection probe anneals to the relevant portion of the amplified DNA and the annealed complex is then captured on a support through the reactive ligand.

In some instances, it is desirable to incorporate a ligand labeled dNTP with the labeled probe in the replication composition to facilitate immobilization of the PCR reaction product on a support and then detection of the immobilized product by means of the labeled probe reagent. For example, a biotin, digoxigenin, or digoxin labeled dNTP could be added to PCR reaction composition. The biotin, digoxigenin, or digoxin incorporated in the PCR product could then be immobilized respectively on to a strepavidin, anti-dixogin or antidigoxigenin antibody support. The immobilized PCR product could then be detected by the presence of the probe label.

### EXAMPLES

### General Methods and Materials Used in the Examples

Materials and methods suitable for the maintenance and growth of bacterial cultures are well known in the art. Techniques suitable for use in the following Examples may be found in Manual of Methods for Genus Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC (1994) or Thomas D. Brock in Biotechnology: A Textbook of Industrial Microbiology, Second Edition (1989) Sinauer Associates, Inc., Sunderland, MA or Bacteriological Analytical Manual. 6th Edition, Association of Official Analytical Chemists, Arlington, VA (1984).

The medium used to grow the pathogenic *E*. *coli* strains and comparative non-target strains was Brain Heart Infusion broth (BHI) obtained from BBL (Becton-Dickenson). Samples of pathogenic *E*. *coli* strains were obtained from cultures grown overnight in BHI broth to approximately 10⁹ cfu/ml. Samples of the comparative non-target strains were enriched in BHI at approximately 10⁹ cfu/ml.

Primers and probes (SEQ ID NOs: 1-12) were prepared by LGC Biosearch Technologies, Inc., 2199 S. McDowell Blvd., Petaluma, CA 94954 USA.

All PCR reactions were carried out using a standard BAX^{®} System (Hygiena Qualicon, Wilmington, DE).

The meaning of abbreviations is as follows: "h" means hour(s), "min" means minute(s), "sec" means second(s), "d" means day(s), "ml" means milliliter(s), "µl" means microliter(s), "cfu" means colony forming unit(s).

### EXAMPLE 1

### Determination of inclusivity/exclusivity of the individual targets via Taqman^{®} assay

Samples of organisms were analyzed to establish inclusivity and exclusivity of individual Taqman^{®} probes disclosed herein. Pure cultures grown overnight achieved cell densities of approximately 1×10⁹ cfu/ml. For inclusivity, independent, bona fide *E*. *coli* O157:H7 isolates were used; for exclusivity non 0157:H7 *E. coli* and other closely related non-target organisms were used to ensure that the assay would discriminate the target organism (0157:H7) from other *E*. *coli* and non-target organisms.

### DNA lysate preparation

Material tested was either food enrichment (ground beef enrichment prepared as described in the BAX^{®} system user guide for the BAX^{®} MP assay) or overnight growth of *E*. *coli* 0157:H7 isolates at 37 °C in BHI media. 5 µl of the material to be tested was added to 200 µl of BAX^{®} lysis reagent (Hygiena Qualicon, Wilmington, DE). The mixture was incubated at 37 °C for 20 minutes, then further incubated at 95 °C for 10 minutes, and finally cooled to 4 °C.

### PCR conditions

2 µl of the DNA lysate as prepared above was used in a PCR reaction that included other reaction components including the primers and probes as listed in TABLE 2.

**TABLE 2**

| **Primers/ Probe** | **Per reaction** | **Dye** | **Quencher** |
|---|---|---|---|
| Forward primer | 150 nM | | |
| Reverse primer | 175 nM | | |
| Probe | 66.7 nM | CALFluor610 | BHQ-2 |

The reagents that were used in the PCR amplification reaction were custom made BAX^{®} System No-Taq Reagent Tablet Kits (Hygiena Qualicon, Wilmington, DE) that contained deoxynucleotides (Roche Diagnostics, Indianapolis, Ind., USA), BSA and surfactants (Sigma-Aldrich, ST. Louis, MO., USA). Additionally the reaction included Go *Taq* DNA Polymerase (Promega, Madison, Wis., USA) and PCR buffer (Hygiena Qualicon, Wilmington, DE).

Amplification and testing was performed on the BAX^{®} Q7 machine (Hygiena Qualicon, Wilmington, DE). The thermal cycling conditions were: 2 minutes at 94 °C, followed by 43 cycles of 94 °C for 10 seconds and 63 °C for 40 seconds, with the fluorescent signal captured during the 63 °C step at each cycle.

### Results

As can be seen in Tables 3-5, below, using individual Taqman^{®} probes, the present methods were able to correctly detect all 0157:H7 isolates and did not detect any non-0157:H7 E. *coli* strains or other non-target organisms.

**TABLE 3**

| **Inclusivity demonstrated towards 242 strains of *E. coli* O157:H7** | | | |
|---|---|---|---|
| **Qualicon Culture Collection Strain #** | **Strain Name** | **Serovar** | **BAX^{®} Result** |
| DD640 | Escherichia coli | O157:H7 | Positive |
| DD641 | Escherichia coli | O157:H7 | Positive |
| DD642 | Escherichia coli | O157:H7 | Positive |
| DD914 | Escherichia coli | O157:H7 | Positive |
| DD915 | Escherichia coli | O157:H7 | Positive |
| DD916 | Escherichia coli | O157:H7 | Positive |
| DD935 | Escherichia coli | O157:H7 | Positive |
| DD1449 | Escherichia coli | O157:H7 | Positive |
| DD1450 | Escherichia coli | O157:H7 | Positive |
| DD1451 | Escherichia coli | O157:H7 | Positive |
| DD1452 | Escherichia coli | O157:H7 | Positive |
| DD1453 | Escherichia coli | O157:H7 | Positive |
| DD1454 | Escherichia coli | O157:H7 | Positive |
| DD1455 | Escherichia coli | O157:H7 | Positive |
| DD1456 | Escherichia coli | O157:H7 | Positive |
| DD1457 | Escherichia coli | O157:H7 | Positive |
| DD1458 | Escherichia coli | O157:H7 | Positive |
| DD1459 | Escherichia coli | O157:H7 | Positive |
| DD1460 | Escherichia coli | O157:H7 | Positive |
| DD1461 | Escherichia coli | O157:H7 | Positive |
| DD1462 | Escherichia coli | O157:H7 | Positive |
| DD1463 | Escherichia coli | O157:H7 | Positive |
| DD1972 | Escherichia coli | O157:H7 | Positive |
| DD1973 | Escherichia coli | O157:H7 | Positive |
| DD1974 | Escherichia coli | O157:H7 | Positive |
| DD1975 | Escherichia coli | O157:H7 | Positive |
| DD1976 | Escherichia coli | O157:H7 | Positive |
| DD1977 | Escherichia coli | O157:H7 | Positive |
| DD1978 | Escherichia coli | O157:H7 | Positive |
| DD1979 | Escherichia coli | O157:H7 | Positive |
| DD1980 | Escherichia coli | O157:H7 | Positive |
| DD1981 | Escherichia coli | O157:H7 | Positive |
| DD1982 | Escherichia coli | O157:H7 | Positive |
| DD1983 | Escherichia coli | O157:H7 | Positive |
| DD1984 | Escherichia coli | O157:H7 | Positive |
| DD1985 | Escherichia coli | O157:H7 | Positive |
| DD1986 | Escherichia coli | O157:H7 | Positive |
| DD1987 | Escherichia coli | O157:H7 | Positive |
| DD1988 | Escherichia coli | O157:H7 | Positive |
| DD1989 | Escherichia coli | O157:H7 | Positive |
| DD1990 | Escherichia coli | O157:H7 | Positive |
| DD1991 | Escherichia coli | O157:H7 | Positive |
| DD5892 | Escherichia coli | O157:H7 | Positive |
| DD5893 | Escherichia coli | O157:H7 | Positive |
| DD5894 | Escherichia coli | O157:H7 | Positive |
| DD5895 | Escherichia coli | O157:H7 | Positive |
| DD5896 | Escherichia coli | O157:H7 | Positive |
| DD5897 | Escherichia coli | O157:H7 | Positive |
| DD5898 | Escherichia coli | O157:H7 | Positive |
| DD6972 | Escherichia coli | O157:H7 | Positive |
| DD6973 | Escherichia coli | O157:H7 | Positive |
| DD7101 | Escherichia coli | O157:H7 | Positive |
| DD8295 | Escherichia coli | O157:H7 | Positive |
| DD8296 | Escherichia coli | O157:H7 | Positive |
| DD8297 | Escherichia coli | O157:H7 | Positive |
| DD8298 | Escherichia coli | O157:H7 | Positive |
| DD8299 | Escherichia coli | O157:H7 | Positive |
| DD8300 | Escherichia coli | O157:H7 | Positive |
| DD8301 | Escherichia coli | O157:H7 | Positive |
| DD8302 | Escherichia coli | O157:H7 | Positive |
| DD8303 | Escherichia coli | O157:H7 | Positive |
| DD8856 | Escherichia coli | O157:H7 | Positive |
| DD8857 | Escherichia coli | O157:H7 | Positive |
| DD8858 | Escherichia coli | O157:H7 | Positive |
| DD8859 | Escherichia coli | O157:H7 | Positive |
| DD8860 | Escherichia coli | O157:H7 | Positive |
| DD8861 | Escherichia coli | O157:H7 | Positive |
| DD8862 | Escherichia coli | O157:H7 | Positive |
| DD8863 | Escherichia coli | O157:H7 | Positive |
| DD8864 | Escherichia coli | O157:H7 | Positive |
| DD8865 | Escherichia coli | O157:H7 | Positive |
| DD8866 | Escherichia coli | O157:H7 | Positive |
| DD8867 | Escherichia coli | O157:H7 | Positive |
| DD8868 | Escherichia coli | O157:H7 | Positive |
| DD8869 | Escherichia coli | O157:H7 | Positive |
| DD8870 | Escherichia coli | O157:H7 | Positive |
| DD8871 | Escherichia coli | O157:H7 | Positive |
| DD8872 | Escherichia coli | O157:H7 | Positive |
| DD8873 | Escherichia coli | O157:H7 | Positive |
| DD8874 | Escherichia coli | O157:H7 | Positive |
| DD9047 | Escherichia coli | O157:H7 | Positive |
| DD9048 | Escherichia coli | O157:H7 | Positive |
| DD9711 | Escherichia coli | O157:H7 | Positive |
| DD9712 | Escherichia coli | O157:H7 | Positive |
| DD9713 | Escherichia coli | O157:H7 | Positive |
| DD9714 | Escherichia coli | O157:H7 | Positive |
| DD9715 | Escherichia coli | O157:H7 | Positive |
| 0010133 | Escherichia coli | O157:H7 | Positive |
| 0010134 | Escherichia coli | O157:H7 | Positive |
| 0010135 | Escherichia coli | O157:H7 | Positive |
| 0010136 | Escherichia coli | O157:H7 | Positive |
| DD10901 | Escherichia coli | O157:H7 | Positive |
| DD10902 | Escherichia coli | O157:H7 | Positive |
| DD10903 | Escherichia coli | O157:H7 | Positive |
| DD10904 | Escherichia coli | O157:H7 | Positive |
| DD10905 | Escherichia coli | O157:H7 | Positive |
| DD10906 | Escherichia coli | O157:H7 | Positive |
| DD10907 | Escherichia coli | O157:H7 | Positive |
| DD10908 | Escherichia coli | O157:H7 | Positive |
| DD10909 | Escherichia coli | O157:H7 | Positive |
| DD10910 | Escherichia coli | O157:H7 | Positive |
| DD10911 | Escherichia coli | O157:H7 | Positive |
| DD10912 | Escherichia coli | O157:H7 | Positive |
| DD10913 | Escherichia coli | O157:H7 | Positive |
| 0010914 | Escherichia coli | O157:H7 | Positive |
| DD10915 | Escherichia coli | O157:H7 | Positive |
| 0010916 | Escherichia coli | O157:H7 | Positive |
| DD10917 | Escherichia coli | O157:H7 | Positive |
| DD10918 | Escherichia coli | O157:H7 | Positive |
| DD10919 | Escherichia coli | O157:H7 | Positive |
| DD10920 | Escherichia coli | O157:H7 | Positive |
| DD10921 | Escherichia coli | O157:H7 | Positive |
| DD12786 | Escherichia coli | O157:H7 | Positive |
| DD12787 | Escherichia coli | O157:H7 | Positive |
| DD12788 | Escherichia coli | O157:H7 | Positive |
| DD12789 | Escherichia coli | O157:H7 | Positive |
| DD12790 | Escherichia coli | O157:H7 | Positive |
| DD12791 | Escherichia coli | O157:H7 | Positive |
| DD12792 | Escherichia coli | O157:H7 | Positive |
| DD12793 | Escherichia coli | O157:H7 | Positive |
| DD12794 | Escherichia coli | O157:H7 | Positive |
| DD12796 | Escherichia coli | O157:H7 | Positive |
| DD12797 | Escherichia coli | O157:H7 | Positive |
| DD12798 | Escherichia coli | O157:H7 | Positive |
| DD12799 | Escherichia coli | O157:H7 | Positive |
| DD12800 | Escherichia coli | O157:H7 | Positive |
| DD12801 | Escherichia coli | O157:H7 | Positive |
| DD12802 | Escherichia coli | O157:H7 | Positive |
| DD12803 | Escherichia coli | O157:H7 | Positive |
| DD12805 | Escherichia coli | O157:H7 | Positive |
| DD12806 | Escherichia coli | O157:H7 | Positive |
| DD12807 | Escherichia coli | O157:H7 | Positive |
| DD12808 | Escherichia coli | O157:H7 | Positive |
| DD12809 | Escherichia coli | O157:H7 | Positive |
| DD12810 | Escherichia coli | O157:H7 | Positive |
| DD12811 | Escherichia coli | O157:H7 | Positive |
| DD12812 | Escherichia coli | O157:H7 | Positive |
| DD12813 | Escherichia coli | O157:H7 | Positive |
| DD12814 | Escherichia coli | O157:H7 | Positive |
| DD12815 | Escherichia coli | O157:H7 | Positive |
| DD12816 | Escherichia coli | O157:H7 | Positive |
| DD12817 | Escherichia coli | O157:H7 | Positive |
| DD12818 | Escherichia coli | O157:H7 | Positive |
| DD12819 | Escherichia coli | O157:H7 | Positive |
| DD12820 | Escherichia coli | O157:H7 | Positive |
| DD12824 | Escherichia coli | O157:H7 | Positive |
| DD12825 | Escherichia coli | O157:H7 | Positive |
| DD12826 | Escherichia coli | O157:H7 | Positive |
| DD12827 | Escherichia coli | O157:H7 | Positive |
| DD12828 | Escherichia coli | O157:H7 | Positive |
| DD12829 | Escherichia coli | O157:H7 | Positive |
| DD12830 | Escherichia coli | O157:H7 | Positive |
| DD12831 | Escherichia coli | O157:H7 | Positive |
| DD12832 | Escherichia coli | O157:H7 | Positive |
| DD12833 | Escherichia coli | O157:H7 | Positive |
| DD12834 | Escherichia coli | O157:H7 | Positive |
| DD12835 | Escherichia coli | O157:H7 | Positive |
| DD12836 | Escherichia coli | O157:H7 | Positive |
| DD12837 | Escherichia coli | O157:H7 | Positive |
| DD12838 | Escherichia coli | O157:H7 | Positive |
| DD12839 | Escherichia coli | O157:H7 | Positive |
| DD12840 | Escherichia coli | O157:H7 | Positive |
| DD12841 | Escherichia coli | O157:H7 | Positive |
| DD12842 | Escherichia coli | O157:H7 | Positive |
| DD12843 | Escherichia coli | O157:H7 | Positive |
| DD12844 | Escherichia coli | O157:H7 | Positive |
| DD12845 | Escherichia coli | O157:H7 | Positive |
| DD12846 | Escherichia coli | O157:H7 | Positive |
| DD12847 | Escherichia coli | O157:H7 | Positive |
| DD12848 | Escherichia coli | O157:H7 | Positive |
| DD12852 | Escherichia coli | O157:H7 | Positive |
| DD12853 | Escherichia coli | O157:H7 | Positive |
| DD12854 | Escherichia coli | O157:H7 | Positive |
| DD12855 | Escherichia coli | O157:H7 | Positive |
| DD12856 | Escherichia coli | O157:H7 | Positive |
| DD12857 | Escherichia coli | O157:H7 | Positive |
| DD12858 | Escherichia coli | O157:H7 | Positive |
| DD12860 | Escherichia coli | O157:H7 | Positive |
| DD12861 | Escherichia coli | O157:H7 | Positive |
| DD12862 | Escherichia coli | O157:H7 | Positive |
| DD12863 | Escherichia coli | O157:H7 | Positive |
| DD12864 | Escherichia coli | O157:H7 | Positive |
| DD12865 | Escherichia coli | O157:H7 | Positive |
| DD12866 | Escherichia coli | O157:H7 | Positive |
| DD12867 | Escherichia coli | O157:H7 | Positive |
| DD12868 | Escherichia coli | O157:H7 | Positive |
| DD12869 | Escherichia coli | O157:H7 | Positive |
| DD12870 | Escherichia coli | O157:H7 | Positive |
| DD12871 | Escherichia coli | O157:H7 | Positive |
| DD12872 | Escherichia coli | O157:H7 | Positive |
| DD12873 | Escherichia coli | O157:H7 | Positive |
| DD12874 | Escherichia coli | O157:H7 | Positive |
| DD12875 | Escherichia coli | O157:H7 | Positive |
| DD12876 | Escherichia coli | O157:H7 | Positive |
| DD12877 | Escherichia coli | O157:H7 | Positive |
| DD12878 | Escherichia coli | O157:H7 | Positive |
| DD12879 | Escherichia coli | O157:H7 | Positive |
| DD12880 | Escherichia coli | O157:H7 | Positive |
| DD12881 | Escherichia coli | O157:H7 | Positive |
| DD12882 | Escherichia coli | O157:H7 | Positive |
| DD12883 | Escherichia coli | O157:H7 | Positive |
| DD12885 | Escherichia coli | O157:H7 | Positive |
| DD12905 | Escherichia coli | O157:H7 | Positive |
| DD13038 | Escherichia coli | O157:H7 | Positive |
| DD13040 | Escherichia coli | O157:H7 | Positive |
| DD13054 | Escherichia coli | O157:H7 | Positive |
| DD13055 | Escherichia coli | O157:H7 | Positive |
| DD13072 | Escherichia coli | O157:H7 | Positive |
| DD13077 | Escherichia coli | O157:H7 | Positive |
| DD13078 | Escherichia coli | O157:H7 | Positive |
| DD13085 | Escherichia coli | O157:H7 | Positive |
| DD13174 | Escherichia coli | O157:H7 | Positive |
| DD13175 | Escherichia coli | O157:H7 | Positive |
| DD13176 | Escherichia coli | O157:H7 | Positive |
| DD13182 | Escherichia coli | O157:H7 | Positive |
| DD13189 | Escherichia coli | O157:H7 | Positive |
| DD13190 | Escherichia coli | O157:H7 | Positive |
| DD13197 | Escherichia coli | O157:H7 | Positive |
| DD13199 | Escherichia coli | O157:H7 | Positive |
| DD13241 | Escherichia coli | O157:H7 | Positive |
| DD13262 | Escherichia coli | O157:H7 | Positive |
| DD13289 | Escherichia coli | O157:H7 | Positive |
| DD13290 | Escherichia coli | O157:H7 | Positive |
| DD13291 | Escherichia coli | O157:H7 | Positive |
| DD13405 | Escherichia coli | O157:H7 | Positive |
| DD13406 | Escherichia coli | O157:H7 | Positive |
| DD13407 | Escherichia coli | O157:H7 | Positive |
| DD13480 | Escherichia coli | O157:H7 | Positive |
| DD13482 | Escherichia coli | O157:H7 | Positive |
| DD13483 | Escherichia coli | O157:H7 | Positive |
| DD13484 | Escherichia coli | O157:H7 | Positive |
| DD13485 | Escherichia coli | O157:H7 | Positive |
| DD13486 | Escherichia coli | O157:H7 | Positive |
| DD13487 | Escherichia coli | O157:H7 | Positive |
| DD13488 | Escherichia coli | O157:H7 | Positive |
| DD13489 | Escherichia coli | O157:H7 | Positive |
| DD13490 | Escherichia coli | O157:H7 | Positive |
| DD13491 | Escherichia coli | O157:H7 | Positive |
| DD13492 | Escherichia coli | O157:H7 | Positive |

**TABLE 4**

| **Exclusivity demonstrated towards 300 strains of non- O157:H7 *E. coli*** | | | |
|---|---|---|---|
| **Qualicon Culture Collection Strain #** | **Strain Name** | **Serovar** | **BAX^{®} Result** |
| DD655 | Escherichia coli | O101:K-:K99 | Negative |
| DD656 | Escherichia coli | O101:K30:K99 | Negative |
| DD683 | Escherichia coli | Serovar unknown | Negative |
| DD743 | Escherichia coli | Serovar unknown | Negative |
| DD1715 | Escherichia coli | O136:HNM | Negative |
| DD1716 | Escherichia coli | O158:H23 | Negative |
| DD1718 | Escherichia coli | O128:H2 | Negative |
| DD1719 | Escherichia coli | O28:HNM | Negative |
| DD1720 | Escherichia coli | O26:HNM | Negative |
| DD1721 | Escherichia coli | O114:H32 | Negative |
| DD1722 | Escherichia coli | O127:HNM | Negative |
| DD1725 | Escherichia coli | O125:H19 | Negative |
| DD1726 | Escherichia coli | O126:H2 | Negative |
| DD1727 | Escherichia coli | O44:H18 | Negative |
| DD1728 | Escherichia coli | O55:HNM | Negative |
| DD1729 | Escherichia coli | O111:HNM | Negative |
| DD1730 | Escherichia coli | O86:H25 | Negative |
| DD1731 | Escherichia coli | O167:H5 | Negative |
| DD1732 | Escherichia coli | O143:HNM | Negative |
| DD1733 | Escherichia coli | O142:H6 | Negative |
| DD1734 | Escherichia coli | O124:H30 | Negative |
| DD1735 | Escherichia coli | O144:HNM | Negative |
| DD1756 | Escherichia coli | O25:H12 | Negative |
| DD1757 | Escherichia coli | O152:HNM | Negative |
| DD1758 | Escherichia coli | O63:HNM | Negative |
| DD1759 | Escherichia coli | O15:H4 | Negative |
| DD1760 | Escherichia coli | O6:H1 | Negative |
| DD1761 | Escherichia coli | O27:HNM | Negative |
| DD1761 | Escherichia coli | O27:HNM | Negative |
| DD1762 | Escherichia coli | O164:HNM | Negative |
| DD1762 | Escherichia coli | O164:HNM | Negative |
| DD1764 | Escherichia coli | O8:H4 | Negative |
| DD1766 | Escherichia coli | O80:H26 | Negative |
| DD1767 | Escherichia coli | O85:H1 | Negative |
| DD1768 | Escherichia coli | O153:H7 | Negative |
| DD1769 | Escherichia coli | O139:H1 | Negative |
| DD1770 | Escherichia coli | O115:H18 | Negative |
| DD1771 | Escherichia coli | O148:H28 | Negative |
| DD1772 | Escherichia coli | O159:H20 | Negative |
| DD1795 | Escherichia coli | O26:HNM | Negative |
| DD1796 | Escherichia coli | O86:HNM | Negative |
| DD1797 | Escherichia coli | O111:HNM | Negative |
| DD1798 | Escherichia coli | O28:HSM | Negative |
| DD1799 | Escherichia coli | O142:H(-) | Negative |
| DD1800 | Escherichia coli | O128:HNM | Negative |
| DD1801 | Escherichia coli | O142:HNM | Negative |
| DD1802 | Escherichia coli | O6:HNM | Negative |
| DD1803 | Escherichia coli | O25:H(-) | Negative |
| DD1804 | Escherichia coli | O124:H(-) | Negative |
| DD1805 | Escherichia coli | O143:HNM | Negative |
| DD1807 | Escherichia coli | O26:H(-) | Negative |
| DD1808 | Escherichia coli | O111:HNM | Negative |
| DD1809 | Escherichia coli | O111:HNM | Negative |
| DD1810 | Escherichia coli | O28:H16 | Negative |
| DD1811 | Escherichia coli | O127:H40 | Negative |
| DD1812 | Escherichia coli | O127:H10 | Negative |
| DD1814 | Escherichia coli | O6:H(-) | Negative |
| DD1817 | Escherichia coli | O29:H(-) | Negative |
| DD1818 | Escherichia coli | O136:H8 | Negative |
| DD1819 | Escherichia coli | O18:HNM | Negative |
| DD1820 | Escherichia coli | O86:H8 | Negative |
| DD1821 | Escherichia coli | O55:H(-) | Negative |
| DD1822 | Escherichia coli | O28:H8,43 | Negative |
| DD1824 | Escherichia coli | O125:HNM | Negative |
| DD1825 | Escherichia coli | O25:H8 | Negative |
| DD1827 | Escherichia coli | O20:HNM | Negative |
| DD1828 | Escherichia coli | O143:HNM | Negative |
| DD1831 | Escherichia coli | O26:H11 | Negative |
| DD1832 | Escherichia coli | O86:HNM | Negative |
| DD1833 | Escherichia coli | O55:H9 | Negative |
| DD1834 | Escherichia coli | O29:H51 | Negative |
| DD1835 | Escherichia coli | O127:H(-) | Negative |
| DD1836 | Escherichia coli | O125:H(-) | Negative |
| DD1839 | Escherichia coli | O15:H(-) | Negative |
| DD1840 | Escherichia coli | O143:HNM | Negative |
| DD1841 | Escherichia coli | 0124 | Negative |
| DD1842 | Escherichia coli | O78:HNM | Negative |
| DD1843 | Escherichia coli | O26:HNM | Negative |
| DD1844 | Escherichia coli | O119:HSM | Negative |
| DD1845 | Escherichia coli | O55:HNM | Negative |
| DD1847 | Escherichia coli | O128:H2,3,6 | Negative |
| DD1848 | Escherichia coli | O126:H27 | Negative |
| DD1849 | Escherichia coli | O27:HNT | Negative |
| DD1851 | Escherichia coli | O20:HNM | Negative |
| DD1852 | Escherichia coli | O152:H2,3,7 | Negative |
| DD1853 | Escherichia coli | O124:H8 | Negative |
| DD1854 | Escherichia coli | O44:HNT | Negative |
| DD1855 | Escherichia coli | O119:H(-) | Negative |
| DD1856 | Escherichia coli | O144:H25 | Negative |
| DD1857 | Escherichia coli | O144:H42 | Negative |
| DD1858 | Escherichia coli | O111:HNM | Negative |
| DD1859 | Escherichia coli | O125:H(-) | Negative |
| DD1860 | Escherichia coli | O126:H27,6 | Negative |
| DD1861 | Escherichia coli | O126:H(-) | Negative |
| DD1862 | Escherichia coli | O27:HNM | Negative |
| DD1864 | Escherichia coli | O15:HNM | Negative |
| DD1865 | Escherichia coli | O144:HNM | Negative |
| DD1866 | Escherichia coli | O18:H(-) | Negative |
| DD1869 | Escherichia coli | O55:H(-) | Negative |
| DD1870 | Escherichia coli | O125:H19 | Negative |
| DD1871 | Escherichia coli | O119:H26 | Negative |
| DD1872 | Escherichia coli | O126:H10 | Negative |
| DD1873 | Escherichia coli | O27:H12 | Negative |
| DD1874 | Escherichia coli | O8:HNM | Negative |
| DD1875 | Escherichia coli | O15:H6 | Negative |
| DD1876 | Escherichia coli | O124:H8,6,2 | Negative |
| DD1878 | Escherichia coli | O152:H6,8,12 | Negative |
| DD1880 | Escherichia coli | O55:H7 | Negative |
| DD1882 | Escherichia coli | O114:H10 | Negative |
| DD1883 | Escherichia coli | O125:HNM | Negative |
| DD1884 | Escherichia coli | O158:H7 | Negative |
| DD1886 | Escherichia coli | O164:HNM | Negative |
| DD1889 | Escherichia coli | O152:H10 | Negative |
| DD1890 | Escherichia coli | O143:HNM | Negative |
| DD1891 | Escherichia coli | O164:HNM | Negative |
| DD1893 | Escherichia coli | O114:H8,10 | Negative |
| DD1894 | Escherichia coli | O114:H(-) | Negative |
| DD1906 | Escherichia coli | O127:H11 | Negative |
| DD1907 | Escherichia coli | O164:HNM | Negative |
| DD1908 | Escherichia coli | O25:H7 | Negative |
| DD1909 | Escherichia coli | O15:HNM | Negative |
| DD1910 | Escherichia coli | O164:HNM | Negative |
| DD1911 | Escherichia coli | O136:H(-) | Negative |
| DD1913 | Escherichia coli | O26:H11 | Negative |
| DD1915 | Escherichia coli | O28:H(-) | Negative |
| DD1918 | Escherichia coli | O164:HNM | Negative |
| DD1919 | Escherichia coli | O8:HNM | Negative |
| DD1922 | Escherichia coli | O63:H6 | Negative |
| DD1924 | Escherichia coli | O136:H16 | Negative |
| DD1925 | Escherichia coli | O152:HNM | Negative |
| DD1927 | Escherichia coli | O111:HNM | Negative |
| DD1930 | Escherichia coli | O158:HNM | Negative |
| DD1931 | Escherichia coli | O127:H(-) | Negative |
| DD1932 | Escherichia coli | O78:HNM | Negative |
| DD1936 | Escherichia coli | O29:H10 | Negative |
| DD1996 | Escherichia coli | O6:H10 | Negative |
| DD1997 | Escherichia coli | O20:HNM | Negative |
| DD1998 | Escherichia coli | O63:HNM | Negative |
| DD1999 | Escherichia coli | O29:HNM | Negative |
| DD2000 | Escherichia coli | O29:H25 | Negative |
| DD2001 | Escherichia coli | O143:HNM | Negative |
| DD2002 | Escherichia coli | O115:HNM | Negative |
| DD2019 | Escherichia coli | O148:H(-) | Negative |
| DD2020 | Escherichia coli | 0139 | Negative |
| DD2026 | Escherichia coli | O153:H(-) | Negative |
| DD2028 | Escherichia coli | 0139 | Negative |
| DD2030 | Escherichia coli | O159:H27 | Negative |
| DD2034 | Escherichia coli | O115:HNM | Negative |
| DD2036 | Escherichia coli | O115:HNM | Negative |
| DD2037 | Escherichia coli | O115:HNM | Negative |
| DD2039 | Escherichia coli | O80:HNM | Negative |
| DD2040 | Escherichia coli | O80:HSM | Negative |
| DD2041 | Escherichia coli | O153:HNM | Negative |
| DD2047 | Escherichia coli | O80:HNM | Negative |
| DD2119 | Escherichia coli | Serovar unknown | Negative |
| DD2130 | Escherichia coli | Serovar unknown | Negative |
| DD2132 | Escherichia coli | Serovar unknown | Negative |
| DD2133 | Escherichia coli | Serovar unknown | Negative |
| DD2152 | Escherichia coli | Serovar unknown | Negative |
| DD2414 | Escherichia coli | Serovar unknown | Negative |
| DD2415 | Escherichia coli | Serovar unknown | Negative |
| DD2429 | Escherichia coli | O112:H18 | Negative |
| DD2430 | Escherichia coli | O11:H10 | Negative |
| DD2431 | Escherichia coli | O167:H5 | Negative |
| DD2432 | Escherichia coli | O165:H(-) | Negative |
| DD2433 | Escherichia coli | O163:H19 | Negative |
| DD2434 | Escherichia coli | O1:H7 | Negative |
| DD2435 | Escherichia coli | O166:H4 | Negative |
| DD2436 | Escherichia coli | O91:HNM | Negative |
| DD2437 | Escherichia coli | O50:H4 | Negative |
| DD2438 | Escherichia coli | O118:HNM | Negative |
| DD2439 | Escherichia coli | O145:HNM | Negative |
| DD2440 | Escherichia coli | O121:H10 | Negative |
| DD2441 | Escherichia coli | O117:H4 | Negative |
| DD2442 | Escherichia coli | O82:HNM | Negative |
| DD2443 | Escherichia coli | O157:H19 | Negative |
| DD2444 | Escherichia coli | O84:H21 | Negative |
| DD2445 | Escherichia coli | O113:H21 | Negative |
| DD2446 | Escherichia coli | O39:HNM | Negative |
| DD2447 | Escherichia coli | O38:H26 | Negative |
| DD2448 | Escherichia coli | O5:H4 | Negative |
| DD2449 | Escherichia coli | O103:H8 | Negative |
| DD2450 | Escherichia coli | O45:H10 | Negative |
| DD2451 | Escherichia coli | O135:HNM | Negative |
| DD2452 | Escherichia coli | O4:H5 | Negative |
| DD2453 | Escherichia coli | O2:H4 | Negative |
| DD2457 | Escherichia coli | O146:H21 | Negative |
| DD2458 | Escherichia coli | O121:HNM | Negative |
| DD2459 | Escherichia coli | O113:H21 | Negative |
| DD2461 | Escherichia coli | O153:H25 | Negative |
| DD2462 | Escherichia coli | O153:H25 | Negative |
| DD2463 | Escherichia coli | O146:H21 | Negative |
| DD2472 | Escherichia coli | O45:H2 | Negative |
| DD2473 | Escherichia coli | O45:H2 | Negative |
| DD2474 | Escherichia coli | O2:H32 | Negative |
| DD2477 | Escherichia coli | O55:H7 | Negative |
| DD2480 | Escherichia coli | O4:HNM | Negative |
| DD2483 | Escherichia coli | O145:HNM | Negative |
| DD2484 | Escherichia coli | O25:HNM | Negative |
| DD2485 | Escherichia coli | O157:H19 | Negative |
| DD2487 | Escherichia coli | O136:HNM | Negative |
| DD2490 | Escherichia coli | O5:HNM | Negative |
| DD2491 | Escherichia coli | O2:H7 | Negative |
| DD2500 | Escherichia coli | O124:HNM | Negative |
| DD2501 | Escherichia coli | O124:HNM | Negative |
| DD2502 | Escherichia coli | O112:HNM | Negative |
| DD2503 | Escherichia coli | O135:HNM | Negative |
| DD2505 | Escherichia coli | O28:HNM | Negative |
| DD2508 | Escherichia coli | O127:HNM | Negative |
| DD2511 | Escherichia coli | O127:HNM | Negative |
| DD2512 | Escherichia coli | O4:HNM | Negative |
| DD2514 | Escherichia coli | O2:H5 | Negative |
| DD2515 | Escherichia coli | O5:HNM | Negative |
| DD2517 | Escherichia coli | O2:H5 | Negative |
| DD2518 | Escherichia coli | O2:H7 | Negative |
| DD2519 | Escherichia coli | O91:H21 | Negative |
| DD2520 | Escherichia coli | O113:H7 | Negative |
| DD2521 | Escherichia coli | O103:H2 | Negative |
| DD2522 | Escherichia coli | O91:HNM | Negative |
| DD2523 | Escherichia coli | O91:HNM | Negative |
| DD2525 | Escherichia coli | O91:H21 | Negative |
| DD2526 | Escherichia coli | O145:HNM | Negative |
| DD2530 | Escherichia coli | O103:H2 | Negative |
| DD2533 | Escherichia coli | O113:H21 | Negative |
| DD3124 | Escherichia coli | O2 | Negative |
| DD3127 | Escherichia coli | O7 | Negative |
| DD3130 | Escherichia coli | O8 | Negative |
| DD3132 | Escherichia coli | O2 | Negative |
| DD3166 | Escherichia coli | O2 | Negative |
| DD3197 | Escherichia coli | O75 | Negative |
| DD3199 | Escherichia coli | O2 | Negative |
| DD3204 | Escherichia coli | O2 | Negative |
| DD3208 | Escherichia coli | 0103 | Negative |
| DD3210 | Escherichia coli | 0103 | Negative |
| DD3785 | Escherichia coli | O111:K58(B4):H- | Negative |
| DD3790 | Escherichia coli | O111:K58(B4):H- | Negative |
| DD4088 | Escherichia coli | Serovar unknown | Negative |
| DD5883 | Escherichia coli | O55:H10 | Negative |
| DD5884 | Escherichia coli | O91:H- | Negative |
| DD5887 | Escherichia coli | O111:H- | Negative |
| DD5901 | Escherichia coli | O5:H- | Negative |
| DD5902 | Escherichia coli | O26:H11 | Negative |
| DD5903 | Escherichia coli | O26:H11 | Negative |
| DD5904 | Escherichia coli | O26:H11 | Negative |
| DD5905 | Escherichia coli | O26:H11 | Negative |
| DD5906 | Escherichia coli | O55:H7 | Negative |
| DD9703 | Escherichia coli | O26:H11 | Negative |
| DD9704 | Escherichia coli | O26:H11 | Negative |
| DD9705 | Escherichia coli | O26:H11 | Negative |
| DD9706 | Escherichia coli | O26:H11 | Negative |
| DD9707 | Escherichia coli | O26:H11 | Negative |
| DD10922 | Escherichia coli | Serovar unknown | Negative |
| DD12804 | Escherichia coli | Serovar unknown | Negative |
| DD12849 | Escherichia coli | Serovar unknown | Negative |
| DD12851 | Escherichia coli | O142:H+ | Negative |
| DD12859 | Escherichia coli | O-:H- | Negative |
| DD12884 | Escherichia coli | Serovar unknown | Negative |
| DD12887 | Escherichia coli | O157:H2 | Negative |
| DD12888 | Escherichia coli | O157:H2 | Negative |
| DD12889 | Escherichia coli | O157:H4 | Negative |
| DD12890 | Escherichia coli | O157:H11 | Negative |
| DD12891 | Escherichia coli | O157:H12 | Negative |
| DD12892 | Escherichia coli | O157:H29 | Negative |
| DD12893 | Escherichia coli | O157:H32 | Negative |
| DD12894 | Escherichia coli | O157:H43 | Negative |
| DD12895 | Escherichia coli | O157:H43 | Negative |
| DD12896 | Escherichia coli | O157:H44 | Negative |
| DD12897 | Escherichia coli | O157:H54 | Negative |
| DD12900 | Escherichia coli | Serovar unknown | Negative |
| DD12901 | Escherichia coli | O-:H- | Negative |
| DD12977 | Escherichia coli | Serovar unknown | Negative |
| DD13041 | Escherichia coli | Serovar unknown | Negative |
| DD13073 | Escherichia coli | Serovar unknown | Negative |
| DD13074 | Escherichia coli | Serovar unknown | Negative |
| DD13076 | Escherichia coli | Serovar unknown | Negative |
| DD13086 | Escherichia coli | Serovar unknown | Negative |
| DD13143 | Escherichia coli | Serovar unknown | Negative |
| DD13196 | Escherichia coli | Serovar unknown | Negative |
| DD13198 | Escherichia coli | Serovar unknown | Negative |
| DD13263 | Escherichia coli | Serovar unknown | Negative |
| DD13292 | Escherichia coli | Serovar unknown | Negative |
| DD13317 | Escherichia coli | Serovar unknown | Negative |
| DD13318 | Escherichia coli | Serovar unknown | Negative |
| DD13319 | Escherichia coli | Serovar unknown | Negative |
| DD13320 | Escherichia coli | Serovar unknown | Negative |
| DD13347 | Escherichia coli | Serovar unknown | Negative |
| DD13348 | Escherichia coli | Serovar unknown | Negative |
| DD13349 | Escherichia coli | O45:H2 | Negative |
| DD13350 | Escherichia coli | O45:H2 | Negative |
| DD13351 | Escherichia coli | O45:H2 | Negative |
| DD13352 | Escherichia coli | O45:H2 | Negative |
| DD13353 | Escherichia coli | O45:H2 | Negative |
| DD13354 | Escherichia coli | O45: | Negative |
| DD13355 | Escherichia coli | O45: | Negative |
| DD13479 | Escherichia coli | Serovar unknown | Negative |
| DD13481 | Escherichia coli | Serovar unknown | Negative |
| DD13493 | Escherichia coli | O104:H4 | Negative |
| DD13832 | Escherichia coli | Serovar unknown | Negative |
| DD13833 | Escherichia coli | Serovar unknown | Negative |
| DD13834 | Escherichia coli | Serovar unknown | Negative |

**TABLE 5**

| **Exclusivity demonstrated towards 185 non-E. coli strains** | | |
|---|---|---|
| **Qualicon Culture Collection Strain #** | **Strain Name** | **BAX^{®} Result** |
| DD227 | Citrobacter koseri | Negative |
| DD373 | Klebsiella pneumoniae | Negative |
| DD375 | Enterobacter cloacae | Negative |
| DD375 | Enterobacter cloacae | Negative |
| DD376 | Enterobacter aerogenes | Negative |
| DD383 | Citrobacter freundii | Negative |
| DD383 | Citrobacter freundii | Negative |
| DD569 | Pseudomonas fluorescens | Negative |
| DD572 | Aeromonas hydrophila | Negative |
| DD576 | Pseudomonas mendocina | Negative |
| DD577 | Pseudomonas stutzeri | Negative |
| DD584 | Salmonella typhi | Negative |
| DD585 | Salmonella typhi | Negative |
| DD586 | Salmonella typhimurium | Negative |
| DD592 | Yersinia enterocolitica | Negative |
| DD610 | Staphylococcus aureus | Negative |
| DD657 | Klebsiella ozaenae | Negative |
| DD659 | Lactococcus lactis | Negative |
| DD700 | Shigella sonnei | Negative |
| DD707 | Salmonella newport | Negative |
| DD715 | Bacillus cereus | Negative |
| DD739 | Salmonella stanley | Negative |
| DD741 | Salmonella gallinarum | Negative |
| DD919 | Salmonella paratyphi | Negative |
| DD966 | Salmonella napoli | Negative |
| DD1074 | Enterobacter cloacae | Negative |
| DD1081 | Shigella boydii | Negative |
| DD1082 | Shigella dysenteriae | Negative |
| DD1085 | Salmonella binza | Negative |
| DD1248 | Salmonella panama | Negative |
| DD1251 | Salmonella Kedougou | Negative |
| DD1329 | Salmonella braenderup | Negative |
| DD1332 | Salmonella anatum | Negative |
| DD1336 | Salmonella thompson | Negative |
| DD1352 | Salmonella agona | Negative |
| DD1356 | Salmonella bredeney | Negative |
| DD1429 | Salmonella anfo | Negative |
| DD1435 | Salmonella brandenburg | Negative |
| DD1469 | Salmonella ealing | Negative |
| DD1482 | Salmonella pullorum | Negative |
| DD1509 | Salmonella bovismorbificans | Negative |
| DD1510 | Salmonella bareilly | Negative |
| DD1521 | Salmonella abaetetuba | Negative |
| DD1523 | Salmonella berkeley | Negative |
| DD1525 | Salmonella betioky | Negative |
| DD1526 | Salmonella austin | Negative |
| DD1530 | Salmonella altendorf | Negative |
| DD1535 | Salmonella brookfield | Negative |
| DD1543 | Salmonella adelaide | Negative |
| DD1547 | Salmonella aberdeen | Negative |
| DD1548 | Salmonella abony | Negative |
| DD1552 | Salmonella alabama | Negative |
| DD2357 | Proteus mirabilis | Negative |
| DD2389 | Hafnia alvei | Negative |
| DD2399 | Yersinia aldovae | Negative |
| DD2552 | Enterococcus faecium | Negative |
| DD2554 | Enterococcus faecalis | Negative |
| DD2558 | Citrobacter freundii | Negative |
| DD2558 | Citrobacter freundii | Negative |
| DD2559 | Citrobacter amalonaticus | Negative |
| DD2559 | Citrobacter amalonaticus | Negative |
| DD2560 | Citrobacter koseri | Negative |
| DD2560 | Citrobacter koseri | Negative |
| DD2561 | Citrobacter koseri | Negative |
| DD2584 | Enterobacter hormaechei | Negative |
| DD2584 | Enterobacter hormaechei | Negative |
| DD2586 | Klebsiella planticola | Negative |
| DD2599 | Pantoea agglomerans | Negative |
| DD2600 | Enterobacter gergoviae | Negative |
| DD2601 | Enterobacter cancerogenus | Negative |
| DD2604 | Enterobacter amnigenus | Negative |
| DD2604 | Enterobacter amnigenus | Negative |
| DD2631 | Vibrio fluvialis | Negative |
| DD2632 | Vibrio vulnificus | Negative |
| DD3097 | Citrobacter freundii | Negative |
| DD3114 | Pantoea agglomerans | Negative |
| DD3785 | Escherichia coli | Negative |
| DD3863 | Salmonella oranienburg | Negative |
| DD3882 | Salmonella broughton | Negative |
| DD3898 | Salmonella thompson | Negative |
| DD3915 | Salmonella haardt | Negative |
| DD3982 | Pseudomonas aeruginosa | Negative |
| DD3984 | Salmonella java | Negative |
| DD4022 | Salmonella enteritidis | Negative |
| DD4036 | Salmonella livingstone | Negative |
| DD4102 | Salmonella saintpaul | Negative |
| DD4656 | Citrobacter freundii | Negative |
| DD4659 | Citrobacter freundii | Negative |
| DD5533 | Salmonella infantis | Negative |
| DD5588 | Hafnia alvei | Negative |
| DD6121 | Prot. Mirabilis | Negative |
| DD6177 | Salmonella arkansas | Negative |
| DD6250 | Salmonella santiago | Negative |
| DD6523 | Klebsiella oxytoca | Negative |
| DD6719 | Escherichia hermanni | Negative |
| DD6735 | Salmonella albany | Negative |
| DD6832 | Shigella sonnei | Negative |
| DD7083 | Serratia marcesens | Negative |
| DD7111 | Salmonella infantis | Negative |
| DD10001 | Cronobacter sakazakii | Negative |
| DD10005 | Cronobacter sakazakii | Negative |
| DD10006 | Cronobacter sakazakii | Negative |
| DD10006 | Enterobacter sakazakii | Negative |
| DD10009 | Cronobacter sakazakii | Negative |
| DD10011 | Cronobacter sakazakii | Negative |
| DD10014 | Cronobacter sakazakii | Negative |
| DD10016 | Cronobacter sakazakii | Negative |
| DD10017 | Cronobacter sakazakii | Negative |
| DD10019 | Cronobacter sakazakii | Negative |
| DD10023 | Cronobacter sakazakii | Negative |
| DD11232 | Vibrio mimicus | Negative |
| DD11330 | Cronobacter sakazakii | Negative |
| DD11334 | Cronobacter sakazakii | Negative |
| DD11335 | Cronobacter sakazakii | Negative |
| DD11336 | Cronobacter sakazakii | Negative |
| DD11338 | Cronobacter sakazakii | Negative |
| DD11339 | Cronobacter sakazakii | Negative |
| DD11340 | Cronobacter sakazakii | Negative |
| DD12720 | Enterobacter sakazakii | Negative |
| DD12720 | Cronobacter sakazakii | Negative |
| DD12721 | Cronobacter sakazakii | Negative |
| DD12722 | Cronobacter sakazakii | Negative |
| DD12723 | Cronobacter sakazakii | Negative |
| DD12725 | Cronobacter sakazakii | Negative |
| DD12726 | Cronobacter sakazakii | Negative |
| DD12727 | Cronobacter sakazakii | Negative |
| DD12728 | Cronobacter sakazakii | Negative |
| DD12729 | Cronobacter sakazakii | Negative |
| DD12731 | Cronobacter sakazakii | Negative |
| DD12732 | Cronobacter sakazakii | Negative |
| DD12733 | Cronobacter sakazakii | Negative |
| DD12760 | Enterobacter cloace | Negative |
| DD12760 | Enterobacter cloace | Negative |
| DD12761 | Cronobacter muytjensii | Negative |
| DD12763 | Cronobacter sakazakii | Negative |
| DD12764 | Cronobacter sakazakii | Negative |
| DD12769 | Enterobacter cloace | Negative |
| DD12777 | Pantoea species | Negative |
| DD12907 | Salmonella heidelberg | Negative |
| DD12914 | Salmonella kentucky | Negative |
| DD12960 | Salmonella senftenberg | Negative |
| DD12968 | Salmonella haardt | Negative |
| DD13005 | Salmonella typhimurium | Negative |
| DD13014 | Salmonella heidelberg | Negative |
| DD13035 | Salmonella Choleraesuis I | Negative |
| DD13056 | Salmonella senftenberg | Negative |
| DD13064 | Salmonella tennessee | Negative |
| DD13066 | Salmonella tennessee | Negative |
| DD13067 | Salmonella havana | Negative |
| DD13068 | Salmonella lexington | Negative |
| DD13069 | Salmonella mbandaka | Negative |
| DD13071 | Salmonella montevideo | Negative |
| DD13075 | Salmonella cubana | Negative |
| DD13079 | Salmonella newport | Negative |
| DD13081 | Salmonella virchow | Negative |
| DD13135 | Enterobacter cloacae | Negative |
| DD13136 | Enterobacter aerogenes | Negative |
| DD13144 | Citrobacter koseri | Negative |
| DD13145 | Pantoea agglomerans | Negative |
| DD13157 | Citrobacter freundii | Negative |
| DD13159 | Enterobacter cloacae | Negative |
| DD13160 | Enterobacter homaechei | Negative |
| DD13161 | Enterobacter asburiae | Negative |
| DD13162 | Enterobacter homaechei | Negative |
| DD13163 | Enterobacter turicensis | Negative |
| DD13164 | Enterobacter helveticus | Negative |
| DD13165 | Enterobacter novel species | Negative |
| DD13166 | Enterobacter helveticus | Negative |
| DD13185 | Enterobacter cloacae | Negative |
| DD13186 | Enterobacter amnigenus | Negative |
| DD13187 | Enterobacter amnigenus | Negative |
| DD13344 | salmonella berta | Negative |
| DD13477 | Citrobacter brakii | Negative |
| DD13499 | Enterobacter turicensis | Negative |
| DD13500 | Enterobacter pulveris | Negative |
| DD13524 | Enterobacter helveticus | Negative |
| DD13525 | Enterobacter pulveris | Negative |
| DD13526 | Enterobacter turicensis | Negative |
| DD13531 | Citrobacter gillenii | Negative |
| DD13532 | Citrobacter murliniae | Negative |
| DD13533 | Citrobacter rodentium | Negative |
| DD13534 | Citrobacter werkmanii | Negative |
| DD13535 | Citrobacter farmeri | Negative |
| DD13630 | Salmonella hadar | Negative |
| DD13901 | Citrobacter+G1 :H186 freundii | Negative |

### Sequence Listing

Number of SEQ ID NOS: 13
SEQ ID NO 1
   LENGTH: 20
   TYPE: DNA
   ORGANISM: Artificial Sequence
   FEATURE:
   OTHER INFORMATION: Primer
SEQUENCE: 1
   ccgcgtgtta cgtccgggct
SEQ ID NO 2
   LENGTH: 20
   TYPE: DNA
   ORGANISM: Artificial Sequence
   FEATURE:
   OTHER INFORMATION: Primer
SEQUENCE: 2
   ttgcataggc gtcaccctga
SEQ ID NO 3
   LENGTH: 20
   TYPE: DNA
   ORGANISM: Artificial Sequence
   FEATURE:
   OTHER INFORMATION: Primer
SEQUENCE:3
   tgaaacccgc gtgttacgtc
SEQ ID NO 4
   LENGTH: 20
   TYPE: DNA
   ORGANISM: Artificial Sequence
   FEATURE:
   OTHER INFORMATION: Primer
SEQUENCE:3
   aggcgtcacc ctgaccgtta
SEQ ID NO 5
   LENGTH: 20
   TYPE: DNA
   ORGANISM: Artificial Sequence
   FEATURE:
   OTHER INFORMATION: Primer
SEQUENCE:3
   gcgtgttacg tccgggctac
SEQ ID NO 6
   LENGTH: 20
   TYPE: DNA
   ORGANISM: Artificial Sequence
   FEATURE:
   OTHER INFORMATION: Primer
SEQUENCE:3
   gggtttgcat aggcgtcacc
   SEQ ID NO 7
   LENGTH: 20
   TYPE: DNA
   ORGANISM: Artificial Sequence
   FEATURE:
   OTHER INFORMATION: Primer
SEQUENCE: 7
   gggctacgtc aagccgaaac
SEQ ID NO 8
   LENGTH: 20
   TYPE: DNA
   ORGANISM: Artificial Sequence
   FEATURE:
   OTHER INFORMATION: Primer
SEQUENCE:3
   gcataggcgt caccctgacc
SEQ ID NO 9
   LENGTH: 21
   TYPE: DNA
   ORGANISM: Artificial Sequence
   FEATURE:
   OTHER INFORMATION: Probe
SEQUENCE:3
   tcaagccgaa acacgaattc c
SEQ ID NO 10
   LENGTH: 22
   TYPE: DNA
   ORGANISM: Artificial Sequence
   FEATURE:
   OTHER INFORMATION: Probe
SEQUENCE: 10
   aattcccctg gagccggtaa aa
SEQ ID NO 11
   LENGTH: 21
   TYPE: DNA
   ORGANISM: Artificial Sequence
   FEATURE:
   OTHER INFORMATION: Probe
SEQUENCE: 11
   gaaacacgaa ttcccctgga g
SEQ ID NO 12
   LENGTH: 21
   TYPE: DNA
   ORGANISM: Artificial Sequence
   FEATURE:
   OTHER INFORMATION: Probe
SEQUENCE: 12
   gccggtaaaa ggagccggta c
SEQ ID NO 13
   LENGTH: 122
   TYPE: DNA
   ORGANISM: Escherichia coli
   FEATURE:
SEQUENCE: 13

TGAAACCCGCGTGTTACGTCCGGGCTACGTCAAGCCGAAACACGAATTCCCCTGG
AGCCGGTAAAAGGAGCCGGTACCACCCTGTGGGTTTATAACGGTCAGGGTGACG
CCTATGCAAACCC

## Claims

1. A method for detecting the presence of *E*. *coli* O157:H7 in a sample, said sample comprising nucleic acids, said method comprising:
(a) providing a reaction mixture comprising (i) primer pair SEQ ID NO:1 and SEQ ID NO:2, (ii) primer pair SEQ ID NO:3 and SEQ ID NO:4, (iii) primer pair SEQ ID NO:5 and SEQ ID NO:6, or (iv) primer pair SEQ ID NO:7 and SEQ ID NO:8, or a combination of primer pairs (i)-(iv);
(b) performing PCR amplification of said nucleic acids of said sample using the reaction mixture of step (a); and
(c) detecting the amplification of step (b), whereby a positive detection of amplification indicates the presence of E. *coli* O157:H7 in the sample.

2. The method of claim 1, wherein said reaction mixture further comprises a nucleic acid probe, preferably wherein said nucleic acid probe comprises SEQ ID NO:9, SEQ ID NO:10, SEQ ID NO:11, SEQ ID NO:12, or a combination thereof.

3. The method of claim 2, wherein said probe further comprises a detectable label and quenching molecule.

4. An isolated polynucleotide consisting of a nucleotide sequence set forth in SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, or SEQ ID NO:12, optionally wherein the isolated polynucleotide is linked to a detectable label.

5. An isolated polynucleotide comprising a primer region having a nucleotide sequence set forth in SEQ ID NO:1, SEQ ID NO:2, SEQ ID NO:3, SEQ ID NO:4, SEQ ID NO:5, SEQ ID NO:6, SEQ ID NO:7, or SEQ ID NO:8 and a probe region having a nucleotide sequence set forth in SEQ ID NO:9, SEQ ID NO:10, SEQ ID NO: 11, or SEQ ID NO:12, wherein said polynucleotide further comprises an 18-carbon non-amplifiable linker joining said primer region to said probe region, and wherein said polynucleotide further comprises a detectable label.

6. A replication composition for use in performance of PCR, comprising:
(a) a primer pair comprising (i) primer pair SEQ ID NO:1 and SEQ ID NO:2, (ii) primer pair SEQ ID NO:3 and SEQ ID NO:4, (iii) primer pair SEQ ID NO:5 and SEQ ID NO:6, or (iv) primer pair SEQ ID NO:7 and SEQ ID NO:8, or a combination of primer pairs (i)-(iv); and
(b) a nucleic acid probe comprising SEQ ID NO:9, SEQ ID NO:10, SEQ ID NO:11, SEQ ID NO:12, or a combination thereof; and
(c) a thermostable DNA polymerase.

7. A kit for detection of *E. coli* O157:H7 in a sample, comprising the replication composition of claim 6.

8. A tablet comprising the replication composition of claim 6.

9. A method for detecting the presence of *E*. *coli* O157:H7 in a sample, said sample comprising nucleic acids, said method comprising:
(a) providing a reaction mixture comprising a suitable primer pair for amplification of a polynucleotide sequence comprising SEQ ID NO:13;
(b) performing PCR amplification of said nucleic acids of said sample using the reaction mixture of step (a); and
(c) detecting the amplification of step (b), whereby a positive detection of amplification indicates the presence of *E. coli* O157:H7 in the sample.

10. The method of claim 9, wherein the polynucleotide sequence of (a) consists of SEQ ID NO:13.

11. The method of claim 9 or 10, wherein said primer pair for amplification of SEQ ID NO:13 is selected from group consisting of primer pair SEQ ID NO:1 and SEQ ID NO:2, primer pair SEQ ID NO:3 and SEQ ID NO:4, primer pair SEQ ID NO:5 and SEQ ID NO:6, primer pair SEQ ID NO:7 and SEQ ID NO:8, and a combination thereof.

12. The method of any one of claims 9-11, wherein said reaction mixture further comprises a nucleic acid probe.

13. The method of claim 12, wherein said nucleic acid probe comprises SEQ ID NO:9, SEQ ID NO:10, SEQ ID NO:11, SEQ ID NO:12, or a combination thereof.

14. The method of claim 12 or 13, wherein said probe further comprises a detectable label and quenching molecule.

15. The method of any one of claims 1-3, or the method of any one of claims 9-14, wherein the sample comprises a food sample or a water sample.

## Patentansprüche

1. Verfahren zum Nachweisen des Vorhandenseins von E. coli O157:H7 in einer Probe, wobei die Probe Nukleinsäuren umfasst, wobei das Verfahren Folgendes umfasst:
(a) Bereitstellen eines Reaktionsgemisches, umfassend (i) Primerpaar SEQ ID NO:1 und SEQ ID NO:2, (ii) Primerpaar SEQ ID NO:3 und SEQ ID NO:4, (iii) Primerpaar SEQ ID NO:5 und SEQ ID NO:6, oder (iv) Primerpaar SEQ ID NO:7 und SEQ ID NO:8 oder eine Kombination der Primerpaare (i)-(iv);
(b) Durchführen einer PCR-Amplifikation der Nukleinsäuren der Probe unter Verwendung des Reaktionsgemisches aus Schritt (a); und
(c) Nachweisen der Amplifikation aus Schritt (b), wobei ein positiver Nachweis der Amplifikation das Vorhandensein von *E. coli* O157:H7 in der Probe angibt.

2. Verfahren nach Anspruch 1, wobei das Reaktionsgemisch weiter eine Nukleinsäuresonde umfasst, wobei vorzugsweise die Nukleinsäuresonde die SEQ ID NO:9, SEQ ID NO:10, SEQ ID NO:11, SEQ ID NO:12 oder eine Kombination davon umfasst.

3. Verfahren nach Anspruch 2, wobei die Sonde weiter ein nachweisbares Markierungs- und Löschmolekül umfasst.

4. Isoliertes Polynukleotid, bestehend aus einer Nukleotidsequenz gemäß SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11 oder SEQ ID NO:12, wobei das isolierte Polynukleotid optional mit einer nachweisbaren Markierung verknüpft ist.

5. Isoliertes Polynukleotid, das eine Primerregion, die eine Nukleotidsequenz gemäß SEQ ID NO:1, SEQ ID NO:2, SEQ ID NO:3, SEQ ID NO:4, SEQ ID NO:5, SEQ ID NO:6, SEQ ID NO:7 oder SEQ ID NO:8 aufweist, und eine Sondenregion, die eine Nukleotidsequenz gemäß SEQ ID NO:9, SEQ ID NO:10, SEQ ID NO:11 oder SEQ ID NO:12 aufweist, umfasst, wobei das Polynukleotid weiter einen nicht amplifizierbaren Linker mit 18 Kohlenstoffatomen umfasst, der die Primerregion mit der Sondenregion verbindet, und wobei das Polynukleotid weiter eine nachweisbare Markierung umfasst.

6. Replikationszusammensetzung zur Verwendung bei der Durchführung einer PCR, die Folgendes umfasst:
(a) ein Primerpaar, umfassend (i) Primerpaar SEQ ID NO:1 und SEQ-ID NO:2, (ii) Primerpaar SEQ ID NO:3 und SEQ-ID NO:4, (iii) Primerpaar SEQ ID NO:5 und SEQ-ID NO:6, oder (iv) Primerpaar SEQ ID NO:7 und SEQ-ID NO:8 oder eine Kombination der Primerpaare (i)-(iv); und
(b) eine Nukleinsäuresonde, umfassend SEQ ID NO:9, SEQ ID NO:10, SEQ ID NO:11, SEQ ID NO:12 oder eine Kombination davon; und
(c) eine thermostabile DNA-Polymerase.

7. Kit zum Nachweis von *E. coli* O157:H7 in einer Probe, die die Replikationszusammensetzung nach Anspruch 6 umfasst.

8. Tablette, die die Replikationszusammensetzung nach Anspruch 6 umfasst.

9. Verfahren zum Nachweisen des Vorhandenseins von E. coli O157:H7 in einer Probe, wobei die Probe Nukleinsäuren umfasst, wobei das Verfahren Folgendes umfasst:
(a) Bereitstellen eines Reaktionsgemisches, das ein geeignetes Primerpaar zur Amplifikation einer Polynukleotidsequenz umfasst, die SEQ ID NO:13 umfasst;
(b) Durchführen einer PCR-Amplifikation der Nukleinsäuren der Probe unter Verwendung des Reaktionsgemisches aus Schritt (a); und
(c) Nachweisen der Amplifikation aus Schritt (b), wobei ein positiver Nachweis der Amplifikation das Vorhandensein von E. coli O157:H7 in der Probe angibt.

10. Verfahren nach Anspruch 9, wobei die Polynukleotidsequenz von (a) aus SEQ ID NO:13 besteht.

11. Verfahren nach Anspruch 9 oder 10, wobei das Primerpaar zur Amplifikation von SEQ ID NO:13 aus der Gruppe ausgewählt ist, bestehend aus Primerpaar SEQ ID NO:1 und SEQ ID NO:2, Primerpaar SEQ ID NO:3 und SEQ ID NO:4, Primerpaar SEQ ID NO:5 und SEQ ID NO:6, Primerpaar SEQ ID NO:7 und SEQ ID NO:8 und einer Kombination davon.

12. Verfahren nach einem der Ansprüche 9-11, wobei das Reaktionsgemisch weiter eine Nukleinsäuresonde umfasst.

13. Verfahren nach Anspruch 12, wobei die Nukleinsäuresonde die SEQ ID NO:9, SEQ ID NO:10, SEQ ID NO:11, SEQ ID NO:12 oder eine Kombination davon umfasst.

14. Verfahren nach Anspruch 12 oder 13, wobei die Sonde weiter ein nachweisbares Markierungs- und Löschmolekül umfasst.

15. Verfahren nach einem der Ansprüche 1-3 oder das Verfahren nach einem der Ansprüche 9-14, wobei die Probe eine Lebensmittelprobe oder eine Wasserprobe umfasst.

## Revendications

1. Procédé de détection de la présence d'*E*. *coli* O157:H7 dans un échantillon, ledit échantillon comprenant des acides nucléiques, ledit procédé comprenant :
(a) la fourniture d'un mélange réactionnel comprenant (i) une paire d'amorces SEQ ID NO:1 et SEQ ID NO:2, (ii) une paire d'amorces SEQ ID NO:3 et SEQ ID NO:4, (iii) une paire d'amorces SEQ ID NO:5 et SEQ ID NO:6, ou (iv) une paire d'amorces SEQ ID NO:7 et SEQ ID NO:8, ou une combinaison de paires d'amorces (i)-(iv) ;
(b) la réalisation d'une amplification par PCR desdits acides nucléiques dudit échantillon à l'aide du mélange réactionnel de l'étape (a) ; et
(c) la détection de l'amplification de l'étape (b), selon lequel une détection positive de l'amplification indique la présence d'*E*. *coli* O157:H7 dans l'échantillon.

2. Procédé selon la revendication 1, dans lequel ledit mélange réactionnel comprend en outre une sonde d'acide nucléique, de préférence dans lequel ladite sonde d'acide nucléique comprend SEQ ID NO:9, SEQ ID NO:10, SEQ ID NO:11, SEQ ID NO:12, ou une combinaison de ceux-ci.

3. Procédé selon la revendication 2, dans lequel ladite sonde comprend en outre un marqueur détectable et une molécule d'extinction.

4. Polynucléotide isolé consistant en une séquence nucléotidique définie dans SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NQ:10, SEQ ID NO:11, ou SEQ ID NO: 12, facultativement dans lequel le polynucléotide isolé est lié à un marqueur détectable.

5. Polynucléotide isolé comprenant une région d'amorce présentant une séquence nucléotidique définie dans SEQ ID NO:1, SEQ ID NO:2, SEQ ID NO:3, SEQ ID NO:4, SEQ ID NO:5, SEQ ID NO:6, SEQ ID NO:7, ou SEQ ID NO:8 et une région de sonde présentant une séquence nucléotidique définie dans SEQ ID NO:9, SEQ ID NO:10, SEQ ID NO:11, ou SEQ ID NO:12, dans lequel ledit polynucléotide comprend en outre un lieur non amplifiable à 18 atomes de carbone reliant ladite région d'amorce à ladite région de sonde, et dans lequel ledit polynucléotide comprend en outre un marqueur détectable.

6. Composition de réplication destinée à être utilisée dans la réalisation d'une PCR, comprenant :
(a) une paire d'amorces comprenant (i) une paire d'amorces SEQ ID NO:1 et SEQ ID NO:2, (ii) une paire d'amorces SEQ ID NO:3 et SEQ ID NO:4, (iii) une paire d'amorces SEQ ID NO:5 et SEQ ID NO:6, ou (iv) une paire d'amorces SEQ ID NO:7 et SEQ ID NO:8, ou une combinaison de paires d'amorces (i)-(iv) ; et
(b) une sonde d'acide nucléique comprenant SEQ ID NO:9, SEQ ID NO:10, SEQ ID NO:11, SEQ ID NO:12, ou une combinaison de ceux-ci ; et
(c) une ADN polymérase thermostable.

7. Kit de détection d'*E*. *coli* O157:H7 dans un échantillon, comprenant la composition de réplication selon la revendication 6.

8. Comprimé comprenant la composition de réplication selon la revendication 6.

9. Procédé de détection de la présence d'*E*. *coli* O157:H7 dans un échantillon, ledit échantillon comprenant des acides nucléiques, ledit procédé comprenant :
(a) la fourniture d'un mélange réactionnel comprenant une paire d'amorces appropriée pour l'amplification d'une séquence polynucléotidique comprenant SEQ ID NO: 13 ;
(b) la réalisation d'une amplification par PCR desdits acides nucléiques dudit échantillon à l'aide du mélange réactionnel de l'étape (a) ; et
(c) la détection de l'amplification de l'étape (b), selon lequel une détection positive de l'amplification indique la présence d'*E*. *coli* O157:H7 dans l'échantillon.

10. Procédé selon la revendication 9, dans lequel la séquence polynucléotidique de (a) consiste en SEQ ID NO:13.

11. Procédé selon la revendication 9 ou 10, dans lequel ladite paire d'amorces pour l'amplification de SEQ ID NO: 13 est choisie dans le groupe consistant en une paire d'amorces SEQ ID NO:1 et SEQ ID NO:2, une paire d'amorces SEQ ID NO:3 et SEQ ID NO:4, une paire d'amorces SEQ ID NO:5 et SEQ ID NO:6, une paire d'amorces SEQ ID NO:7 et SEQ ID NO:8, et une combinaison de celles-ci.

12. Procédé selon l'une quelconque des revendications 9-11, dans lequel ledit mélange réactionnel comprend en outre une sonde d'acide nucléique.

13. Procédé selon la revendication 12, dans lequel ladite sonde d'acide nucléique comprend SEQ ID NO:9, SEQ ID NO:10, SEQ ID NO:11, SEQ ID NO:12, ou une combinaison de ceux-ci.

14. Procédé selon la revendication 12 ou 13, dans lequel ladite sonde comprend en outre un marqueur détectable et une molécule d'extinction.

15. Procédé selon l'une quelconque des revendications 1-3, ou procédé selon l'une quelconque des revendications 9-14, dans lequel l'échantillon comprend un échantillon alimentaire ou un échantillon d'eau.
